# EUROPEAN PATENT APPLICATION

(11) **EP 4 279 092 A1**
(43) Date of publication of application: **22.11.2023**
(21) Application number: 22173871.9
(22) Date of filing: 17.05.2022
(51) Int. Cl.: A61K 51/10, A61P 35/00, C07K 16/30

(54) **RADIOPHARMACEUTICAL COMPLEXES TARGETING PROSTATE-SPECIFIC MEMBRANE ANTIGEN**

(71) Applicant: Bayer AG, 51373 Leverkusen (DE); Bayer AS, 0283 Oslo (NO)
(72) Inventor: The designation of the inventor has not yet been filed
(74) Representative: BIP Patents

(57) **Abstract**

The present invention relates to tissue-targeting compounds. In particular, the present invention relates to tissue-targeting compounds of formula (I) comprising a monoclonal antibody or an antigen-binding fragment thereof having binding affinity for the prostate-specific membrane antigen (PSMA).

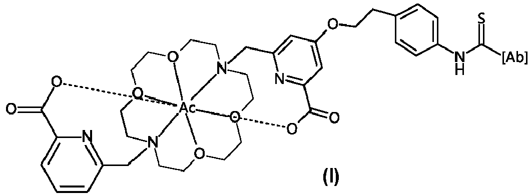

## Description

### FIELD OF THE INVENTION

The present invention relates to tissue-targeting compounds. In particular, the present invention relates to tissue-targeting compounds comprising a monoclonal antibody or an antigen-binding fragment thereof having binding affinity for the prostate-specific membrane antigen (PSMA).

Further, the present invention relates to a process for the preparation of said tissue-targeting compounds and to their use in the treatment of disease, particularly against hyperproliferative diseases, in particular against cancer.

### BACKGROUND OF THE INVENTION

Specific cell killing can be essential for the successful treatment of a variety of diseases in mammalian subjects. Typical examples of this are the treatment of malignant diseases such as sarcomas and carcinomas. However, the selective elimination of certain cell types can also play a key role in the treatment of other diseases, especially hyperplastic and neoplastic diseases.

The most common methods of selective treatment are currently surgery and external beam irradiation. Targeted radionuclide therapy is, however, a promising and developing area with the potential to deliver highly cytotoxic radiation specifically to cell types associated with disease. The most common forms of radiopharmaceuticals currently authorized for use in humans employ beta-emitting and/or gamma-emitting radionuclides. There has, however, been some interest in the use of alpha-emitting radionuclides in therapy because of their potential for more potent cell killing.

The radiation range of typical alpha emitters in physiological surroundings is generally less than 100 micrometers, the equivalent of only a few cell diameters. This makes these sources well suited for the treatment of tumors, including micro-metastases, because they have the range to reach neighboring cells within a tumor but if they are well targeted then little of the radiated energy will pass beyond the target cells. Thus, not every cell need be targeted but damage to surrounding healthy tissue may be minimized (see Feinendegen et al., Radiat. Res. 148:195-201 (1997)). In contrast, a beta particle has a range of 1 mm or more in water (see Wilbur, Antibody Immunocon. Radiopharm. 4: 85-96 (1991)).

The energy of alpha-particle radiation is high in comparison with that carried by beta particles, gamma rays and X-rays, typically being 5-8 MeV, or 5 to 10 times that of a beta particle and 20 or more times the energy of a gamma ray. Thus, this deposition of a large amount of energy over a very short distance gives alpha-radiation an exceptionally high linear energy transfer (LET), high relative biological efficacy (RBE) and low oxygen enhancement ratio (OER) compared to gamma and beta radiation (see Hall, "Radiobiology for the radiologist", Fifth edition, Lippincott Williams & Wilkins, Philadelphia PA, USA, 2000). This explains the exceptional cytotoxicity of alpha emitting radionuclides and also imposes stringent demands on the biological targeting of such isotopes and upon the level of control and study of alpha emitting radionuclide distribution which is necessary in order to avoid unacceptable side effects.

So far, with regards to the application in radioimmunotherapy the main attention has been focused on 211At, 213Bi and 225Ac and these three nuclides have been explored in clinical immunotherapy trials. However, although targeted radiotherapy has been practiced for some time using macrocyclic complexes of radionuclides, the compounds currently in use, which typically employ the macrocyclic chelator DOTA (2,2',2",2"'-(1,4,7,10-Tetraazacyclododecane-1,4,7,10-tetrayl)tetraacetic acid) are lacking in stability, leading to the dissociation of the radionuclide from the chelating macrocycle, which results in a reduced selectivity and activity to the targeted tissue and an increased toxicity to the non-targeted tissue.

For example, it has been reported that a high chelator-to-antigen (CAR) of 10 has been used in a pre-clinical study using a one-step radiolabeling procedure of a DOTA antibody chelator conjugate (ACC) (William F. Maguire, Michael R. McDevitt, Peter M. Smith-Jones, David A. Scheinberg. "Efficient one-step radiolabeling of monoclonal antibodies to high specific activity with Actinium-225 for alpha-particle radioimmunotherapy of cancer", J. Nucl. Med. 2014; 55(9): 1492-1498). However, after 2h labeling at 37 °C, quantitative labeling was not achieved which subsequentially requires a purification step to remove non-labeled Ac-225. This is in contrast to the tissue-targeting compounds of the present invention, in which Ac-225 can be chelated quantitatively at room temperature after one hour leading to complexes with significantly lower CAR ratios (<1).

While the use of larger macrocycles has led to more stable complexation of especially radionuclides of larger size, there is a need for the development of targeting compounds with improved stability, selectivity and efficacy, especially in the field of hyperproliferative diseases such as cancer. For some types of cancer, especially prostate cancer, patients initially respond to therapy but eventually develop resistances (Swami U, McFarland TR, Nussenzveig R, Agarwal N. Advanced Prostate Cancer: Treatment Advances and Future Directions. Trends Cancer 2020;6(8):702-15 doi 10.1016/j.trecan.2020.04.010). Thus, there is a need for new agents capable of selectively targeting specific cells and cell types in malignant diseases which are highly effective and reduce, mitigate and/or avoid the prevalence of resistances. In particular, there is a need to provide new therapeutic agents for the treatment of cancer and especially prostate cancer, which is the most frequent cancer in men (Mattiuzzi C, Lippi G. Current Cancer Epidemiology. J Epidemiol Glob Health 2019;9(4):217-22 doi 10.2991/jegh.k.191008.001). The prostate-specific membrane antigen (PSMA) encoded by the FOLH1 (folate hydrolase 1) gene, is highly expressed in prostate cancer cells, making it a suitable target for radiotherapy.

It has surprisingly been found that the tissue-targeting compounds of formula (I) of the present invention show advantageous properties with regard to their stability, activity, efficacy and selectivity. In particular, the tissue-targeting compounds of formula (I) of the present invention show high values for the immunoreactive fraction (IRF) at low chelator-to-antigen (CAR) ratios and high monomeric purity, thus leading to a higher fraction of radionuclide-labelled compound having affinity for PSMA. Further, the tissue-targeting compounds of formula (I) of the present invention show a favorable biodistribution in vivo and low accumulation in the liver, which is a sign of reduced dissociation of the radionuclide from the chelating moiety and therefore of increased stability. In contrast to previously reported examples in the literature, the tissue-targeting compounds of formula (I) of the present invention can be labelled with the radionuclide under very mild conditions and at reduced temperatures (i.e., room temperature), thereby reducing antibody denaturation and the amount of non-PSMA binding fraction in the final product. Thus, the tissue-targeting compounds of formula (I) of the present invention show a better tumor to liver ratio and can therefore treat disease more effectively and selectively, reducing damage to non-targeted tissue while maintaining a high potency against cancer cells in the targeted tissue.

### SUMMARY OF THE INVENTION

The present invention relates to tissue-targeting compounds of formula (I) 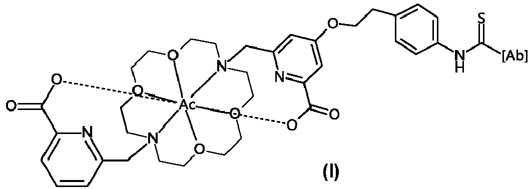 wherein [Ab] is a monoclonal antibody or an antigen-binding fragment thereof having binding affinity for the prostate-specific membrane antigen (PSMA).

### DEFINITIONS

The term "comprising" when used in the specification includes "consisting of".

If within the present text any item is referred to as "as mentioned herein", it means that it may be mentioned anywhere in the present text.

In the context of the present invention, "tissue targeting" is used herein to indicate that the substance in question (i.e. a tissue-targeting compound, a tissue-targeting actinium complex and/or a tissue-targeting moiety, particularly when in the form of a tissue-targeting complex as described herein), serves to localize itself (and particularly to localize any conjugated actinium complex) preferentially to at least one tissue site at which its presence (e.g. to deliver a radioactive decay) is desired. Thus, a tissue-targeting compound, complex, group or moiety serves to provide greater localization to at least one desired site in the body of a subject following administration to that subject in comparison with the concentration of an equivalent complex not having the targeting moiety. The targeting moiety in the present case will be preferably selected to bind specifically to cell-surface receptors associated with cancer cells or other receptors associated with the tumor microenvironment.

In the context of the present invention, the term "PSMA" refers to the prostate-specific membrane antigen encoded by the FOLH1 (folate hydrolase 1) gene (GeneID: 2346).

The compounds of formula (I) of the present invention comprise a chelating moiety comprising a complexed actinium atom (Ac). In the context of the present invention, the term "Ac" means an ion of at least one alpha-emitting actinium isotope. Preferably, the alpha-emitting actinium isotope is 225Ac. Preferably, the ion of at the least one alpha-emitting actinium isotope is ²²⁵Ac having a triple positive charge, i.e., ²²⁵Ac³⁺. Thus, in the context of the present invention, the term "Ac" preferably, but not exclusively, means²²⁵Ac³⁺.

In the compounds of formula (I) of the present invention, the actinium (Ac) atom is shown as being complexed to four oxygen and two nitrogen atoms of the macrocyclic ring as well as to two carboxylate groups. While this is the assumed complexation pattern for the actinium atom, this depiction includes all possible and conceivable cases in which one or more of the bonds is not present, e.g., where the actinium atom is not bound to all heteroatoms of the macrocyclic ring, or to the carboxylate groups, etc.

In the context of the present invention, the term IRF refers to the Immunoreactive Fraction i.e., the fraction of the labelled product (i.e., the compounds of formula (I) of the present invention) which is capable of binding to the target (i.e., PSMA). The Lindmo assay (Lindmo T., et al. (1984) "Determination of the immunoreactive fraction of radiolabeled monoclonal antibodies by linear extrapolation to binding at infinite antigen excess." J. Immunol. Methods. 72, 77-89) is the most commonly used method for assessing the immunoreactive fraction.

In the context of the present invention, the term CAR refers to the Chelator-to-Antigen Ratio, which is a measure of the specific activity of the radiolabeled compound (e.g., the compounds of formula (I) of the present invention).

The terms "polypeptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer. Unless otherwise indicated, a particular polypeptide sequence also implicitly encompasses conservatively modified variants thereof.

Amino acids may be referred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "antibody", as used herein, is intended to refer to immunoglobulin molecules including, but not limited to, full-length antibodies and monovalent antibodies. "Full-length antibodies" are preferably comprised of four polypeptide chains, two heavy (H) chains and two light (L) chains which are typically inter-connected by disulfide bonds. Each heavy chain is comprised of a heavy chain variable region (abbreviated herein as VH) and a heavy chain constant region. The heavy chain constant region can comprise e.g., three domains CH1, CH2 and CH3. Each light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4. "Monovalent antibodies" as used herein are preferably comprised of three polypeptide chains, two heavy (H) chains and one light (L) chain which are typically inter-connected by disulfide bonds. One heavy chain is comprised of a heavy chain variable region (abbreviated herein as VH) and a heavy chain constant region. The heavy chain constant region can comprise e.g., three domains CH1, CH2 and CH3. The other heavy chain is comprised of a heavy chain constant region only. The light chain is comprised of a light chain variable region (abbreviated herein as VL) and a light chain constant region. The light chain constant region is comprised of one domain (CL). The VH and VL regions can be further subdivided into regions of hypervariability, termed complementarity determining regions (CDR), interspersed with regions that are more conserved, termed framework regions (FR). Each VH and VL is typically composed of three CDRs and up to four FRs arranged from amino-terminus to carboxy-terminus e.g., in the following order: FR1, CDR1, FR2, CDR2, FR3, CDR3, FR4.

As used herein, the term "Complementarity Determining Regions" (CDRs; e.g., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (e.g. about residues 24-34 (L1), 50-56 (L2) and 89-97 (L3) in the light chain variable domain and 31-35 (H1), 50-65 (H2) and 95-102 (H3) in the heavy chain variable domain; (Kabat et al., Sequences of Proteins of Immunolological Interest, 5th Ed. Public Health Service, National Institutes of Health, Bethesda, MD. (1991)) and/or those residues from a "hypervariable loop" (e.g. about residues 26-32 (L1), 50-52 (L2) and 91-96 (L3) in the light chain variable domain and 26- 32 (H1), 53-55 (H2) and 96-101 (H3) in the heavy chain variable domain (Chothia and Lesk; J Mol Biol 196: 901-917 (1987)). In some instances, a complementarity determining region can include amino acids from both a CDR region defined according to Kabat and a hypervariable loop.

Depending on the amino acid sequence of the constant domain of their heavy chains, intact antibodies can be assigned to different "classes". There are five major classes of intact antibodies: IgA, IgD, IgE, IgG, and IgM, and several of these maybe further divided into "subclasses" (isotypes), e.g., IgG1, IgG2, IgG3, IgG4, IgA1, and IgA2. A preferred class of immunoglobulins for use in the present invention is IgG.

The heavy-chain constant domains that correspond to the different classes of antibodies are called [alpha], [delta], [epsilon], [gamma], and [mu], respectively. The subunit structures and three-dimensional configurations of different classes of immunoglobulins are well known. As used herein antibodies are conventionally known antibodies and functional fragments thereof.

A "functional fragment" or "antigen-binding antibody fragment" of an antibody/immunoglobulin hereby is defined as a fragment of an antibody/immunoglobulin (e.g., a variable region of an IgG) that retains the antigen-binding region. An "antigen-binding region" of an antibody typically is found in one or more hyper variable region(s) of an antibody, e.g., the CDR1, -2, and/or -3 regions; however, the variable "framework" regions can also play an important role in antigen binding, such as by providing a scaffold for the CDRs.

"Functional fragments", "antigen-binding antibody fragments", or "antibody fragments" of the invention include but are not limited to Fab, Fab', Fab'-SH, F(ab')₂, and Fv fragments; diabodies; single domain antibodies (DAbs), linear antibodies; single-chain antibody molecules (scFv); and multi-specific, such as bi- and tri-specific, antibodies formed from antibody fragments (C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). An antibody other than a "multi-specific" or "multi-functional" antibody is understood to have each of its binding sites identical. The F(ab')₂ or Fab may be engineered to minimize or completely remove the intermolecular disulfide interactions that occur between the CH1 and CL domains.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal Lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

Variants of the antibodies or antigen-binding antibody fragments contemplated in the invention are molecules in which the binding activity of the antibody or antigen-binding antibody fragment is maintained.

"Binding proteins" contemplated in the invention are for example antibody mimetics, such as Affibodies, Adnectins, Anticalins, DARPins, Avimers, Nanobodies (reviewed by Gebauer M. et al., Curr. Opinion in Chem. Biol. 2009; 13:245-255; Nuttall S.D. et al., Curr. Opinion in Pharmacology 2008; 8:608-617).

A "human" antibody or antigen-binding fragment thereof is hereby defined as one that is not chimeric (e.g., not "humanized") and not from (either in whole or in part) a non-human species. A human antibody or antigen-binding fragment thereof can be derived from a human or can be a synthetic human antibody. A "synthetic human antibody" is defined herein as an antibody having a sequence derived, in whole or in part, in silico from synthetic sequences that are based on the analysis of known human antibody sequences. In silico design of a human antibody sequence or fragment thereof can be achieved, for example, by analyzing a database of human antibody or antibody fragment sequences and devising a polypeptide sequence utilizing the data obtained there from. Another example of a human antibody or antigen-binding fragment thereof is one that is encoded by a nucleic acid isolated from a library of antibody sequences of human origin (e.g., such library being based on antibodies taken from a human natural source). Examples of human antibodies include antibodies as described in Söderlind et al., Nature Biotech. 2000, 18:853-856.

A "humanized antibody" or humanized antigen-binding fragment thereof is defined herein as one that is (i) derived from a non-human source (e.g., a transgenic mouse which bears a heterologous immune system), which antibody is based on a human germline sequence; (ii) where amino acids of the framework regions of a non-human antibody are partially exchanged to human amino acid sequences by genetic engineering or (iii) CDR-grafted, wherein the CDRs of the variable domain are from a non-human origin, while one or more frameworks of the variable domain are of human origin and the constant domain (if any) is of human origin.

A "chimeric antibody" or antigen-binding fragment thereof is defined herein as one, wherein the variable domains are derived from a non-human origin and some or all constant domains are derived from a human origin.

The term "monoclonal antibody" as used herein refers to an antibody obtained from a population of substantially homogeneous antibodies, i.e., the individual antibodies comprising the population are identical except for possible mutations, e.g., naturally occurring mutations, that may be present in minor amounts. Thus, the term "monoclonal" indicates the character of the antibody as not being a mixture of discrete antibodies. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins. The term "monoclonal" is not to be construed as to require production of the antibody by any particular method. The term monoclonal antibody specifically includes chimeric, humanized and human antibodies.

An "isolated" antibody is one that has been identified and separated from a component of the cell that expressed it. Contaminant components of the cell are materials that would interfere with diagnostic or therapeutic uses of the antibody, and may include enzymes, hormones, and other proteinaceous or non-proteinaceous solutes.

An "isolated" nucleic acid is one that has been identified and separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

As used herein, an antibody "binds specifically to", is "specific to/for" or "specifically recognizes" an antigen of interest, e.g. a tumor-associated polypeptide antigen target or an antigen-binding polypeptide target (as e.g. an antigen-binding antibody), is one that binds the antigen-target with sufficient affinity such that the antibody is useful as a therapeutic agent in targeting a cell or tissue expressing the antigen or one that binds an antigen-binding polypeptide target with sufficient affinity such that the antibody is useful as a reversal agent to neutralize the therapeutic activity of this antigen-binding polypeptide (e.g. an antigen-binding antibody) and does not significantly cross-react with other proteins or does not significantly cross-react with proteins other than orthologs and variants (e.g. mutant forms, splice variants, or proteolytically truncated forms) of the aforementioned target. The term "specifically recognizes" or "binds specifically to" or is "specific to/for" a particular polypeptide or an epitope on a particular polypeptide target as used herein can be exhibited, for example, by an antibody, or antigen-binding fragment thereof, having a monovalent K_{D} for the antigen of less than about 10⁻⁴ M, alternatively less than about 10⁻⁵ M, alternatively less than about 10⁻⁶ M, alternatively less than about 10⁻⁷ M, alternatively less than about 10⁻⁸ M, alternatively less than about 10⁻⁹ M, alternatively less than about 10⁻¹⁰ M, alternatively less than about 10⁻¹¹ M, alternatively less than about 10⁻¹² M, or less. An antibody "binds specifically to," is "specific to/for" or "specifically recognizes" an antigen if such antibody is able to discriminate between such antigen and one or more reference antigen(s). In its most general form, "specific binding", "binds specifically to", is "specific to/for" or "specifically recognizes" is referring to the ability of the antibody to discriminate between the antigen of interest and an unrelated antigen, as determined, for example, in accordance with one of the following methods. Such methods comprise, but are not limited to, surface plasmon resonance (SPR), Western blots, ELISA-, RIA-, ECL-, IRMA-tests and peptide scans. For example, a standard ELISA assay can be carried out. The scoring may be carried out by standard color development (e.g., secondary antibody with horseradish peroxidase and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative is more than 5-fold, 10-fold, 50-fold, and preferably more than 100-fold. Typically, determination of binding specificity is performed by using not a single reference antigen, but a set of about three to five unrelated antigens, such as milk powder, BSA, transferrin or the like.

"Binding affinity" or "affinity" refers to the strength of the total sum of non-covalent interactions between a single binding site of a molecule and its binding partner. Unless indicated otherwise, as used herein, "binding affinity" refers to intrinsic binding affinity which reflects a 1:1 interaction between members of a binding pair (e.g., an antibody and an antigen). The dissociation constant "K_{D}" is commonly used to describe the affinity between a molecule (such as an antibody) and its binding partner (such as an antigen) i.e., how tightly a ligand binds to a particular protein. Ligand-protein affinities are influenced by non-covalent intermolecular interactions between the two molecules. Affinity can be measured by common methods known in the art, including those described herein. In one embodiment, the "K_{D}" or "K_{D} value" according to this invention is measured by using surface plasmon resonance assays using suitable devices including but not limited to Biacore instruments like Biacore T100, Biacore T200, Biacore 2000, Biacore 4000, a Biacore 3000 (GE Healthcare Biacore, Inc.), or a ProteOn XPR36 instrument (Bio-Rad Laboratories, Inc.).

"Antibody-dependent cell-mediated cytotoxicity" or "ADCC" refers to a form of cytotoxicity in which secreted Ig bound onto Fc gamma receptors (FcyRs) present on certain cytotoxic cells (e.g. NK cells, neutrophils, and macrophages) enable these cytotoxic effector cells to bind specifically to an antigen-bearing target cell and subsequently kill the target cell e.g. with cytotoxins. To assess ADCC activity of an antibody of interest, an in vitro ADCC assay, such as that described in US Patent No. 5,500,362 or 5,821,337 or U.S. Patent No. 6,737,056 (Presta), may be performed. Useful effector cells for such assays include PBMC and NK cells.

"Complement dependent cytotoxicity" or "CDC" refers to the lysis of a target cell in the presence of complement. Activation of the classical complement pathway is initiated by the binding of the first component of the complement system (C1q) to antibodies (of the appropriate subclass), which are bound to their cognate antigen. To assess complement activation, a CDC assay, e.g., as described in Gazzano-Santoro et al., J. Immunol. Methods 202: 163 (1996), may be performed. Polypeptide variants with altered Fc region amino acid sequences (polypeptides with a variant Fc region) and increased or decreased C1q binding are described, e.g., in US Patent No. 6,194,551 B1 and WO 1999/51642.

"Percent (%) sequence identity" with respect to a reference polynucleotide or polypeptide sequence, respectively, is defined as the percentage of nucleic acid or amino acid residues, respectively, in a candidate sequence that are identical with the nucleic acid or amino acid residues, respectively, in the reference polynucleotide or polypeptide sequence, respectively, after aligning the sequences and introducing gaps, if necessary, to achieve the maximum percent sequence identity. Conservative substitutions are not considered as part of the sequence identity. Preferred are un-gapped alignments. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full-length of the sequences being compared.

Substantial sequence identity/similarity may be taken as having a sequence similarity/identity of at least 80% to the complete sequences and/or at least 90% to the specific binding regions (e.g., the CDR regions). Preferable sequence similarity or more preferably identity may be at least 92%, 95%, 97%, 98% or 99%. Sequence similarity and/or identity may be determined using the "BestFit" program of the Genetics Computer Group Version 10 software package from the University of Wisconsin. The program uses the local had algorithm of Smith and Waterman with default values: Gap creation penalty=8, Gap extension penalty=2, Average match=2.912, average mismatch 2.003.

The terms "polynucleotide" or "nucleic acid", as used interchangeably herein, refer to chains of nucleotides of any length, and include DNA and RNA. The nucleotides can be deoxyribonucleotides, ribonucleotides, modified nucleotides or bases, and/or their analogs, or any substrate that can be incorporated into a chain by DNA or RNA polymerase. A polynucleotide may comprise modified nucleotides, such as methylated nucleotides and their analogs.

"Sequence homology" indicates the percentage of amino acids that either is identical or that represent conservative amino acid substitutions.

In addition, the nucleic acid sequences encoding variable regions of the heavy and/or light chains can be converted, for example, to nucleic acid sequences encoding full-length antibody chains, Fab fragments, or to scFv. The VL- or VH-encoding DNA fragment can be operatively linked, (such that the amino acid sequences encoded by the two DNA fragments are in-frame) to another DNA fragment encoding, for example, an antibody constant region or a flexible linker. The sequences of human heavy chain and light chain constant regions are known in the art (see e.g., Kabat, E. A., el al. (1991) Sequences of Proteins of Immunological Interest, Fifth Edition, U.S. Department of Health and Human Services, NIH Publication No. 91-3242) and DNA fragments encompassing these regions can be obtained by standard PCR amplification.

To create a polynucleotide sequence that encodes a scFv, the VH- and VL-encoding nucleic acids can be operatively linked to another fragment encoding a flexible linker such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., Nature (1990) 348:552-554).

To express the antibodies, antigen binding fragments thereof or variants thereof standard recombinant DNA expression methods can be used (see, for example, Goeddel; Gene Expression Technology. Methods in Enzymology 185, Academic Press, San Diego, Calif. (1990)). For example, DNA encoding the desired polypeptide can be inserted into an expression vector which is then transfected into a suitable host cell. Suitable host cells are prokaryotic and eukaryotic cells. Examples for prokaryotic host cells are e.g., bacteria, examples for eukaryotic hosts cells are yeasts, insects and insect cells, plants and plant cells, transgenic animals, or mammalian cells. In some embodiments, the DNAs encoding the heavy and light chains are inserted into separate vectors. In other embodiments, the DNA encoding the heavy and light chains is inserted into the same vector. It is understood that the design of the expression vector, including the selection of regulatory sequences is affected by factors such as the choice of the host cell, the level of expression of protein desired and whether expression is constitutive or inducible.

Useful expression vectors for bacterial use are constructed by inserting a DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include but are not limited to E. coli, Bacillus subtilis, Salmonella typhimurium and various species within the genera Pseudomonas, Streptomyces, and Staphylococcus.

Bacterial vectors may be, for example, bacteriophage-, plasmid- or phagemid-based. These vectors can contain a selectable marker and a bacterial origin of replication derived from commercially available plasmids typically containing elements of the well-known cloning vector pBR322 (ATCC 37017). Following transformation of a suitable host strain and growth of the host strain to an appropriate cell density, the selected promoter is de-repressed/induced by appropriate means (e.g., temperature shift or chemical induction) and cells are cultured for an additional period. Cells are typically harvested by centrifugation, disrupted by physical or chemical means, and the resulting crude extract retained for further purification.

In bacterial systems, a number of expression vectors may be advantageously selected depending upon the use intended for the protein being expressed. For example, when a large quantity of such a protein is to be produced, for the generation of antibodies or to screen peptide libraries, for example, vectors which direct the expression of high levels of fusion protein products that are readily purified may be desirable.

Antibodies of the present invention or antigen-binding fragments thereof or variants thereof include naturally purified products, products of chemical synthetic procedures, and products produced by recombinant techniques from a prokaryotic host, including, for example, E. coli, Bacillus subtilis, Salmonella typhimurium and various species within the genera Pseudomonas, Streptomyces, and Staphylococcus, preferably, from E. coli cells.

Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. Expression of the antibodies may be constitutive or regulated (e.g. inducible by addition or removal of small molecule inductors such as Tetracyclin in conjunction with Tet system). For further description of viral regulatory elements, and sequences thereof, see e.g., U.S. 5,168,062 by Stinski, U.S. 4,510,245 by Bell et al. and U.S. 4,968,615 by Schaffner et al.. The recombinant expression vectors can also include origins of replication and selectable markers (see e.g., U.S. 4,399,216, 4,634,665 and U.S. 5,179,017). Suitable selectable markers include genes that confer resistance to drugs such as G418, puromycin, hygromycin, blasticidin, zeocin/bleomycin or methotrexate or selectable marker that exploit auxotrophies such as Glutamine Synthetase (Bebbington et al., Biotechnology (N Y). 1992 Feb;10(2):169-75), on a host cell into which the vector has been introduced. For example, the dihydrofolate reductase (DHFR) gene confers resistance to methotrexate, neo gene confers resistance to G418, the bsd gene from Aspergillus terreus confers resistance to blasticidin, puromycin N-acetyl-transferase confers resistance to puromycin, the Sh ble gene product confers resitance to zeocin, and resistance to hygromycin is conferred by the E. coli hygromycin resistance gene (hyg or hph). Selectable markers like DHFR or Glutamine Synthetase are also useful for amplification techniques in conjunction with MTX and MSX.

Transfection of the expression vector into a host cell can be carried out using standard techniques such as electroporation, nucleofection, calcium-phosphate precipitation, lipofection, polycation-based transfection such as polyethlylenimine (PEI)-based transfection and DEAE-dextran transfection.

Suitable mammalian host cells for expressing the antibodies, antigen binding fragments thereof or variants thereof provided herein include but are not limited to Chinese Hamster Ovary (CHO cells) such as CHO-K1, CHO-S, CHO-K1SV [including dhfr- CHO cells, described in Urlaub and Chasin, (1980) Proc. Natl. Acad. Sci. USA 77:4216-4220 and Urlaub et al., Cell. 1983 Jun;33(2):405-12, used with a DHFR selectable marker, e.g., as described in R. J. Kaufman and P. A. Sharp (1982) Mol. Biol. 159:601-621; and other knockout cells exemplified in Fan et al., Biotechnol Bioeng. 2012 Apr;109(4):1007-15], NS0 myeloma cells, COS cells, HEK293 cells, HKB11 cells, BHK21 cells, CAP cells, EB66 cells, and SP2 cells.

Expression might also be transient or semi-stable in expression systems such as HEK293, HEK293T, HEK293-EBNA, HEK293E, HEK293-6E, HEK293-Freestyle, HKB11, Expi293F, 293EBNALT75, CHO Freestyle, CHO-S, CHO-K1, CHO-K1SV, CHOEBNALT85, CHOS-XE, CHO-3E7 or CAP-T cells (for instance Durocher et al., Nucleic Acids Res. 2002 Jan 15;30(2):E9).

In some embodiments, the expression vector is designed such that the expressed protein is secreted into the culture medium in which the host cells are grown. The antibodies, antigen binding fragments thereof or variants thereof can be recovered from the culture medium using standard protein purification methods.

Antibodies of the invention or antigen-binding fragments thereof or variants thereof can be recovered and purified from recombinant cell cultures by well-known methods including, but not limited to ammonium sulfate or ethanol precipitation, acid extraction, Protein A chromatography, Protein G chromatography, anion or cation exchange chromatography, phospho-cellulose chromatography, hydrophobic interaction chromatography, affinity chromatography, hydroxylapatite chromatography, mixed mode chromatography and lectin chromatography. High performance liquid chromatography ("HPLC") can also be employed for purification. See, e.g., Colligan, Current Protocols in Immunology, or Current Protocols in Protein Science, John Wiley & Sons, NY, N.Y., (1997-2001), e.g., Chapters 1, 4, 6, 8, 9, 10, each entirely incorporated herein by reference.

Antibodies of the present invention or antigen-binding fragments thereof or variants thereof include naturally purified products, products of chemical synthetic procedures, and products produced by recombinant techniques from an eukaryotic host, including, for example, yeast, higher plant, insect and mammalian cells. Depending upon the host employed in a recombinant production procedure, the antibody of the present invention can be glycosylated or can be non-glycosylated. Such methods are described in many standard laboratory manuals, such as Sambrook, supra, Sections 17.37-17.42; Ausubel, supra, Chapters 10, 12, 13, 16, 18 and 20.

In preferred embodiments, the antibody is purified (1) to greater than 95% by weight of antibody as determined e.g. by the Lowry method, UV-Vis spectroscopy or by by SDS-Capillary Gel electrophoresis (for example on a Caliper LabChip GXII, GX 90 or Biorad Bioanalyzer device), and in further preferred embodiments more than 99% by weight, (2) to a degree sufficient to obtain at least 15 residues of N-terminal or internal amino acid sequence, or (3) to homogeneity by SDS-PAGE under reducing or non-reducing conditions using Coomassie blue or, preferably, silver stain. Isolated naturally occurring antibody includes the antibody in situ within recombinant cells since at least one component of the antibody's natural environment will not be present. Ordinarily, however, isolated antibody will be prepared by at least one purification step.

As used in the context of the present invention, the term "Ac225-Macropa-Pelgifatamab" refers to the following compound: 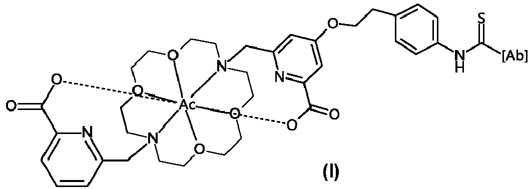 wherein [Ab] is the anti-PSMA monoclonal antibody Pelgifatamab or an antigen-binding fragment thereof which is bound to the rest of the molecule via a terminal amino group of a Lysine residue of the monoclonal antibody or antigen-binding fragment thereof.

As used in the context of the present invention, the term "Ac225-DOTA-Pelgifatamab" refers to the following compound: 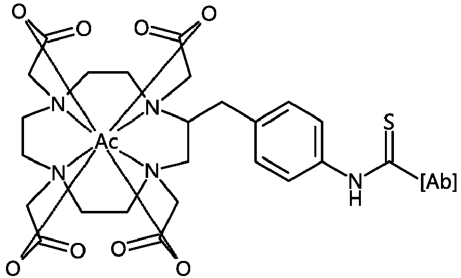 wherein [Ab] is the anti-PSMA monoclonal antibody Pelgifatamab or an antigen-binding fragment thereof which is bound to the rest of the molecule via a terminal amino group of a Lysine residue of the monoclonal antibody or antigen-binding fragment thereof.

### DESCRIPTION OF THE INVENTION

In accordance with a first aspect, the present invention relates to a tissue-targeting compound of formula (I) 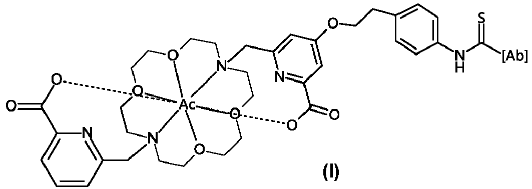 wherein [Ab] is a monoclonal antibody or an antigen-binding fragment thereof having binding affinity for the prostate-specific membrane antigen (PSMA).

In a further embodiment, the present invention relates to a tissue-targeting compound of formula (I) 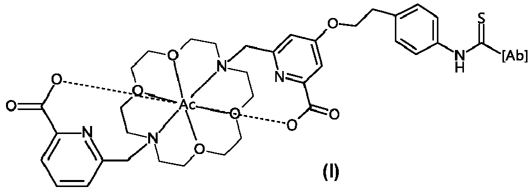 wherein [Ab] is a monoclonal antibody or an antigen-binding fragment thereof having binding affinity for the prostate-specific membrane antigen (PSMA), wherein [Ab] is bound to the rest of the molecule via a terminal amino group of a Lysine residue of the monoclonal antibody or antigen-binding fragment thereof.

In a further embodiment, the present invention relates to a tissue-targeting compound of formula (I) 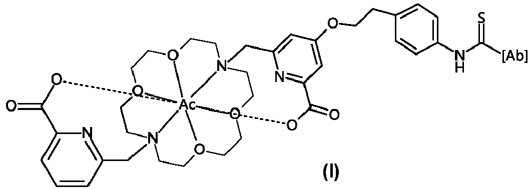 wherein [Ab] is a monoclonal antibody or an antigen-binding fragment thereof having binding affinity for the prostate-specific membrane antigen (PSMA) selected from Pelgifatamab, J591 and TLX592.

In a further embodiment, the present invention relates to a tissue-targeting compound of formula (I) 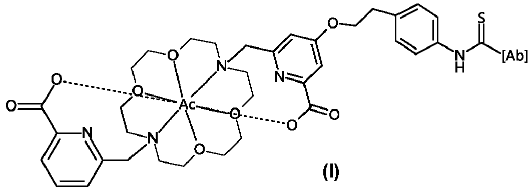 wherein [Ab] is an anti-PSMA monoclonal antibody selected from Pelgifatamab, J591 and TLX592 or an antigen-binding fragment thereof.

In a further embodiment, the present invention relates to a tissue-targeting compound of formula (I) 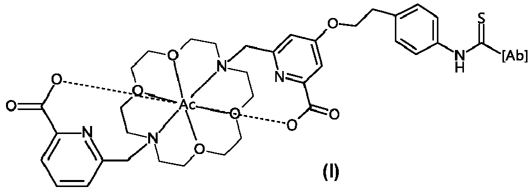 wherein [Ab] is a monoclonal antibody or an antigen-binding fragment thereof having binding affinity for the prostate-specific membrane antigen (PSMA) selected from Pelgifatamab and J591.

In a further embodiment, the present invention relates to a tissue-targeting compound of formula (I) 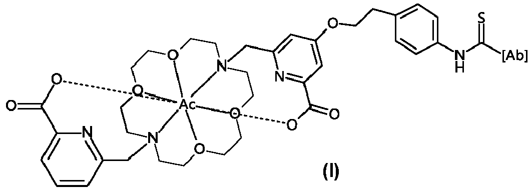 wherein [Ab] is an anti-PSMA monoclonal antibody selected from Pelgifatamab and J591 or an antigen-binding fragment thereof.

In a further embodiment, the present invention relates to a tissue-targeting compound of formula (I) 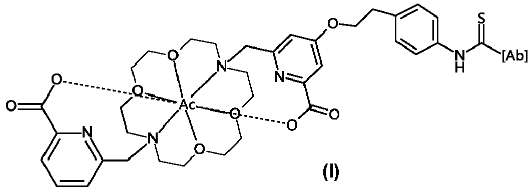 wherein [Ab] is the monoclonal antibody or an antigen-binding fragment thereof having binding affinity for the prostate-specific membrane antigen (PSMA) J591.

In a further embodiment, the present invention relates to a tissue-targeting compound of formula (I) 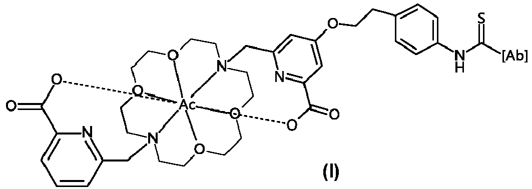 wherein [Ab] is the anti-PSMA monoclonal antibody J591 or an antigen-binding fragment thereof.

In a further embodiment, the present invention relates to a tissue-targeting compound of formula (I) 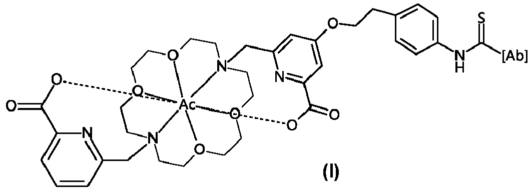 wherein [Ab] is the monoclonal antibody or an antigen-binding fragment thereof having binding affinity for the prostate-specific membrane antigen (PSMA) Pelgifatamab.

In a further embodiment, the present invention relates to a tissue-targeting compound of formula (I) 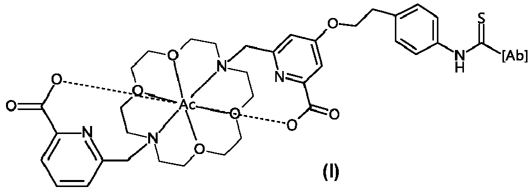 wherein [Ab] is the monoclonal antibody or an antigen-binding fragment thereof having binding affinity for the prostate-specific membrane antigen (PSMA) Pelgifatamab, wherein Pelgifatamab or an antigen-binding fragment thereof is bound to the rest of the molecule via a terminal amino group of a Lysine residue of the monoclonal antibody or antigen-binding fragment thereof.

In a further embodiment, the present invention relates to a tissue-targeting compound of formula (I) 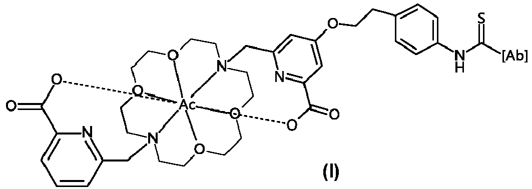 wherein [Ab] is the anti-PSMA monoclonal antibody Pelgifatamab or an antigen-binding fragment thereof.

In a further embodiment, the present invention relates to a tissue-targeting compound of formula (I) 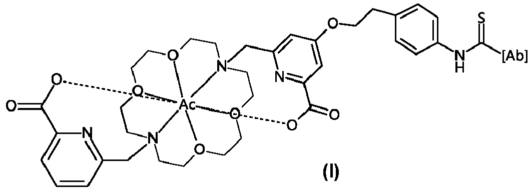 wherein [Ab] is the anti-PSMA monoclonal antibody Pelgifatamab or an antigen-binding fragment thereof, wherein Pelgifatamab or an antigen-binding fragment thereof is bound to the rest of the molecule via a terminal amino group of a Lysine residue of the monoclonal antibody or antigen-binding fragment thereof.

In a further embodiment, the present invention relates to a tissue-targeting compound of formula (I) 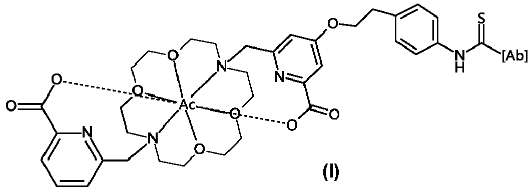 wherein [Ab] is the anti-PSMA monoclonal antibody Pelgifatamab or an antigen-binding fragment thereof comprising a heavy chain nucleotide sequence with sequence similarity or identity with SEQ ID NO. 1 and a light chain nucleotide sequence with sequence similarity or identity with SEQ ID NO. 2.

In a further embodiment, the present invention relates to a tissue-targeting compound of formula (I) 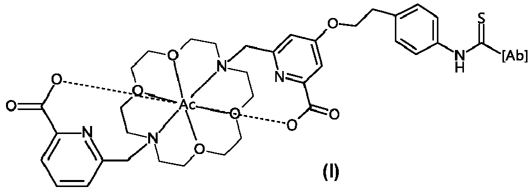 wherein [Ab] is the anti-PSMA monoclonal antibody Pelgifatamab or an antigen-binding fragment thereof comprising a heavy chain sequence according to SEQ ID NO. 1, and a light chain sequence according to SEQ ID NO. 2.

In a further embodiment, the present invention relates to a tissue-targeting compound of formula (I) 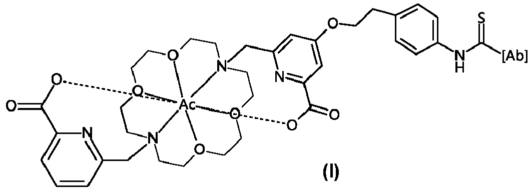 wherein [Ab] is the anti-PSMA monoclonal antibody Pelgifatamab or an antigen-binding fragment thereof comprising a heavy chain sequence according to SEQ ID NO. 1, and a light chain sequence according to SEQ ID NO. 2, wherein Pelgifatamab or an antigen-binding fragment thereof is bound to the rest of the molecule via a terminal amino group of a Lysine residue of the monoclonal antibody or antigen-binding fragment thereof.

In a further embodiment, the present invention relates to a tissue-targeting compound of formula (I) 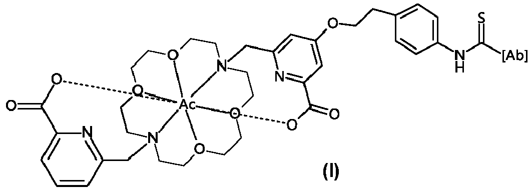 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10.

In a further embodiment, the present invention relates to a tissue-targeting compound of formula (I) 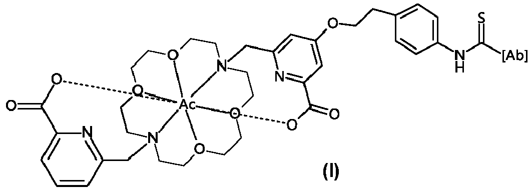 wherein [Ab] is the anti-PSMA monoclonal antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10, wherein Pelgifatamab or an antigen-binding fragment thereof is bound to the rest of the molecule via a terminal amino group of a Lysine residue of the monoclonal antibody or antigen-binding fragment thereof

In a further embodiment, the present invention relates to a tissue-targeting compound of formula (I) 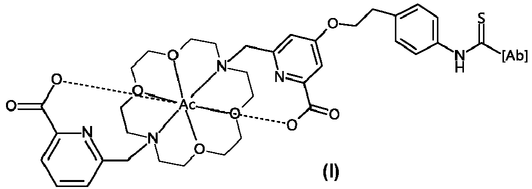 wherein [Ab] is the anti-PSMA monoclonal antibody Pelgifatamab or an antigen-binding fragment thereof comprising a heavy chain sequence according to SEQ ID NO. 1:
SEQ ID NO. 1 - Heavy Chain 
caggtgcagctcgtggaatcgggggggggcgtggtccagccggggcggtcgctccgcctctcgtgcgcagcctcgggcttcgc
cttcagccgctacggcatgcactgggtccgccaggcgcccggcaagggcctggagtgggtcgcggtcatctggtacgacgggt
ccaacaagtattatgcagactccgtcaagggtcggttcaccatcagccgggacaactccaagaacacccagtacctgcagatg
aacagccttcgagcggaggacaccgcggtctactactgcgcccggggcggggatttcctgtactactactactatggcatggacg
 
tctggggccagggtaccaccgtgaccgtatcctccgcatccactaagggcccgtccgttttcccgctggcaccttcgtcgaagagc
acgtccgggggcacagccgcgctgggctgcctcgtgaaggactactttccggagcccgtcaccgtgtcatggaactcgggcgc
gctcacgtccggcgtccacacgttcccggccgtattgcagagctcgggactctactccctgagctcggtcgtcaccgtgccctcctc
gagcctcggcacccagacctacatctgcaacgtgaaccacaaaccctccaatacgaaggtggacaagcgcgtcgaaccgaa
gtcgtgcgacaagacccacacgtgcccgccgtgcccggcaccggagctgctcggcgggccctcggtcttcctgttcccgccgaa
gcccaaggacaccctgatgatctcgcgcaccccggaggtcacatgcgtggtcgtggacgtgtcccacgaagacccggaggtc
aagttcaactggtacgtcgacggcgtcgaggtccacaacgcgaagaccaagccgcgggaggagcagtacaacagcacgta
ccgcgtcgtttccgtcctcaccgtcttgcaccaggactggctgaacggcaaggagtacaagtgtaaggtgtccaacaaggccttg
cccgcccccattgagaagaccatcagcaaggccaagggccagccccgcgagccgcaggtctacaccctgcccccgtcgcgc
gaagaaatgacgaagaaccaggtctccctgacctgcctcgtcaaggggttctacccgtcggacatcgcagtggagtgggagtc
gaacggccagcccgagaataactacaagacgacccccccagtcctggactccgacggcagcttcttcctttactccaagctgac
cgtcgacaagtcgcggtggcagcagggcaacgtcttctcgtgctcggtgatgcacgaggcgctgcacaaccactacacccaga
agtccctctcgctgtcgccgggcaagtga,
 and a light chain sequence according to SEQ ID NO. 2:
SEQ ID NO. 2 - Light Chain

gacatccagatgacccagagccccagctccctgtccgcgtcggtcggagaccgggtcaccatcacctgccgcgcgtcgcagg
gcatctcgaactacctggcgtggtaccagcagaagaccggcaaggtgccgaagttccttatctacgaggcctctacgctccagtc
gggagtcccgtcgcggttcagcgggggtggctccgggaccgacttcacgctgaccatctcgagtctccagccggaggacgtcg
cgacctattactgtcagaactacaactcggccccccttcaccttcggcccgggcaccaaggtcgacatcaagcgcaccgtcgcgg
cgccgtccgtcttcatcttccccccgtcggacgagcagctcaagtccggcaccgcctcggtcgtgtgcctgctcaacaacttctacc
cccgagaggcaaaggtgcagtggaaggtcgacaacgccctgcagtccggcaactcgcaggagagcgtcacggagcagga
cagcaaggacagtacctacagcctctcctccaccctgaccctgagtaaagccgactacgagaagcacaaggtgtacgcgtgc
gaagtcacccaccagggcctctcgtcccccgtgaccaagtccttcaaccggggcgagtgctga.

In a further embodiment, the present invention relates to a tissue-targeting compound of formula (I) 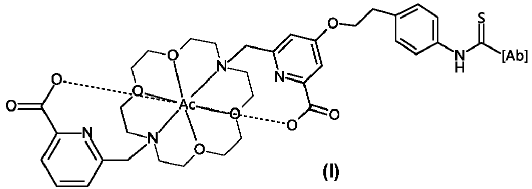 wherein [Ab] is the anti-PSMA monoclonal antibody Pelgifatamab or an antigen-binding fragment thereof comprising a heavy chain amino acid sequence with sequence similarity or identity with SEQ ID NO. 3 and a light chain amino acid sequence with sequence similarity or identity with SEQ ID NO. 4.

In a further embodiment, the present invention relates to a tissue-targeting compound of formula (I) 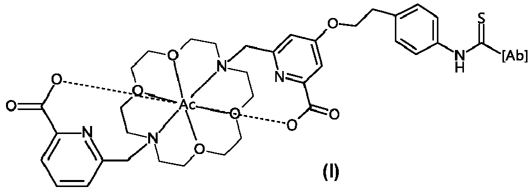 wherein [Ab] is the anti-PSMA monoclonal antibody Pelgifatamab or an antigen-binding fragment thereof comprising a heavy chain amino acid sequence according to SEQ ID NO. 3, and a light chain amino acid sequence according to SEQ ID NO. 4.

In a further embodiment, the present invention relates to a tissue-targeting compound of formula (I) 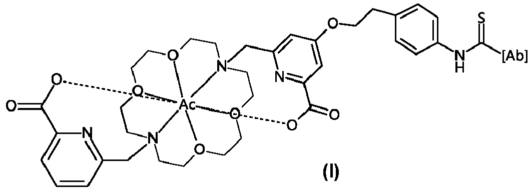 wherein [Ab] is the anti-PSMA monoclonal antibody Pelgifatamab or an antigen-binding fragment thereof comprising a heavy chain amino acid sequence according to SEQ ID NO. 3:
SEQ ID NO. 3 - Heavy Chain 
QVQLVESGGG VVQPGRSLRL SCAASGFAFS RYGMHWVRQA PGKGLEWVAV
IWYDGSNKYY ADSVKGRFTI SRDNSKNTQY LQMNSLRAED TAVYYCARGG
DFLYYYYYGM DVWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
 
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PG(K)
 and a light chain amino acid sequence according to SEQ ID NO. 4:
SEQ ID NO. 4 - Light Chain

DIQMTQSPSS LSASVGDRVT ITCRASQGIS NYLAWYQQKT GKVPKFLIYE
ASTLQSGVPS RFSGGGSGTD FTLTISSLQP EDVATYYCQN YNSAPFTFGP
GTKVDIKRTV AAPSVFIFPP SDEQLKSGTA SVVCLLNNFY PREAKVQWKV
DNALQSGNSQ ESVTEQDSKD STYSLSSTLT LSKADYEKHK VYACEVTHQG
LSSPVTKSFN RGEC.

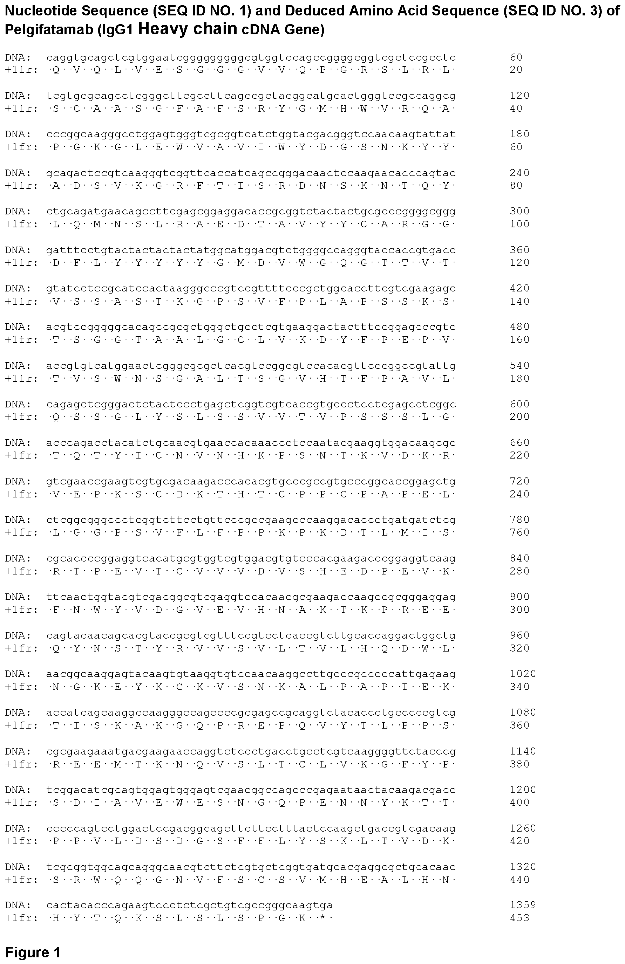

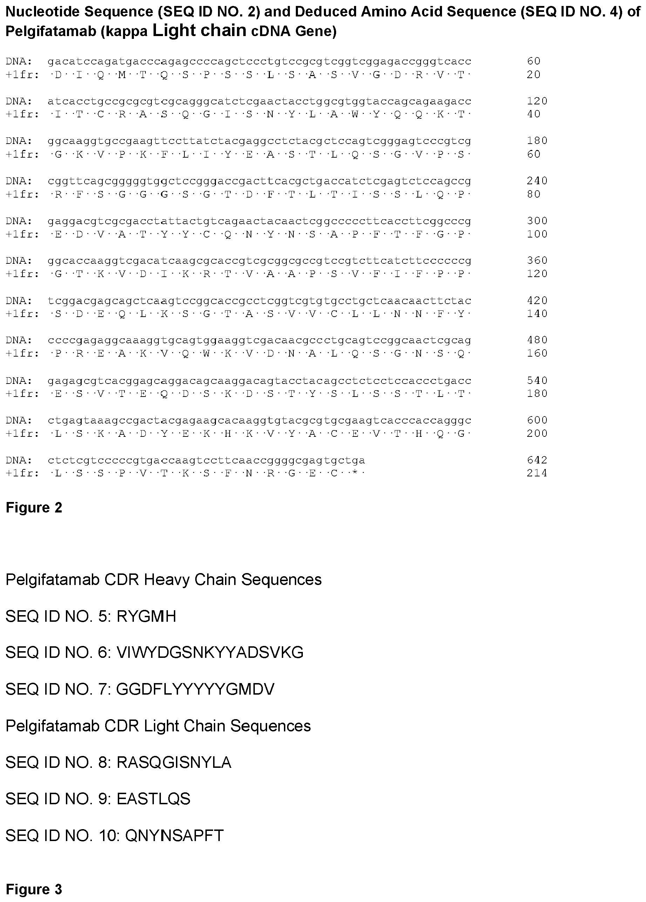

In a further embodiment, the present invention relates to a tissue-targeting compound of formula (I) 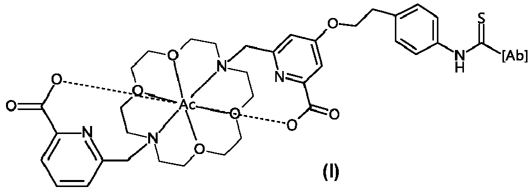 wherein [Ab] is the anti-PSMA monoclonal antibody Pelgifatamab or an antigen-binding fragment thereof comprising a heavy chain nucleotide sequence and/or a deduced amino acid sequence with similarity or identity with the sequences shown in Figure 1 and a light chain nucleotide sequence and/or a deduced amino acid sequence with similarity or identity with the sequences shown in Figure 2.

In a further embodiment, the present invention relates to a tissue-targeting compound of formula (I) 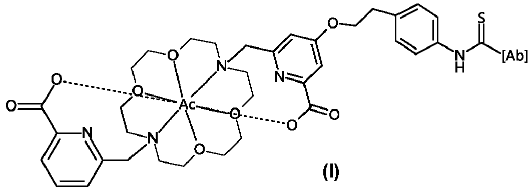 wherein [Ab] is the anti-PSMA monoclonal antibody Pelgifatamab or an antigen-binding fragment thereof comprising a heavy chain nucleotide sequence and/or a deduced amino acid sequence according to the sequences shown in Figure 1 and a light chain nucleotide sequence and/or a deduced amino acid sequence according to the sequences shown in Figure 2.

In a further embodiment, the present invention relates to a tissue-targeting compound of formula (I) 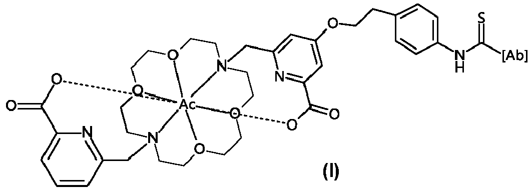 wherein [Ab] is the anti-PSMA monoclonal antibody Pelgifatamab or an antigen-binding fragment thereof comprising at least the three CDR heavy chain sequences and the three CDR light chain sequences according to the sequences shown in Figure 3.

In a further embodiment, the present invention relates to a tissue-targeting compound of formula (I) 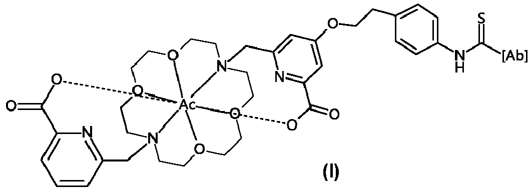 wherein [Ab] is the anti-PSMA monoclonal antibody Pelgifatamab or an antigen-binding fragment thereof comprising at least the three CDR heavy chain sequences and the three CDR light chain sequences according to the sequences shown in Figure 3, wherein Pelgifatamab or an antigen-binding fragment thereof is bound to the rest of the molecule via a terminal amino group of a Lysine residue of the monoclonal antibody or antigen-binding fragment thereof

The tissue-targeting compounds of formula (I) of the present invention comprise a chelating moiety capable of binding actinium. Preferably, the chelating moiety comprises an 18-membered chelating moiety. Preferably, the chelating moiety comprises the macrocyclic chelator N,N'-bis[(6-carboxy-2-pyridil)methyl]-4,13-diaza-18-crown-6 (macropa).

The macrocyclic chelator macropa has been described, for example, in Thiele et al., Angew. Chem. Int. Ed. 2017, 56(46), 14712 and references therein. Macropa chelators and derivatives thereof have also been described in WO2018/183906 and WO2020/106886 and references therein.

The actinium-complexating chelating moiety preferably terminates in an isothiocyanate or carboxylic acid moiety in order to facilitate its coupling with an anti-PSMA antibody or an antigen-binding fragment thereof. When the macropa chelator is used, one of the pyridine rings of the macropa chelator is typically substituted so that it can be coupled to the anti-PSMA antibody or an antigen-binding fragment thereof. This can be a simple functional group (e.g., a carboxylic acid -COOH) or a more complex chemical structure, as long as it is capable of being coupled to an anti-PSMA antibody or an antigen-binding fragment thereof. In particular, the substituent on the pyridine ring of the macropa chelator preferably terminates in an isothiocyanate or carboxylic acid moiety.

The anti-PSMA antibody or an antigen-binding fragment thereof is preferably coupled to the rest of the molecule forming the tissue-targeting compounds of the present invention through a Lysine residue of the antibody. Thus, the actinium-complexating chelating moiety and the anti-PSMA antibody or the antigen-binding fragment thereof can be coupled via an amide or thiourea moiety. When using the preferred macrocyclic chelator macropa, a terminal isothiocyanate or carboxylic acid moiety is coupled with a terminal amino group of a Lysine residue in the anti-PSMA antibody or an antigen-binding fragment thereof, leading to the formation of a thiourea or an amide group, respectively. Thus, the preferred actinium-complexating chelating moiety comprising a macropa chelator is attached to an anti-PSMA antibody or an antigen-binding fragment thereof via a thiourea or an amide bond derived from a terminal amino group of a Lysine residue of said anti-PSMA antibody or an antigen-binding fragment thereof.

Preferred antibodies or antigen-binding fragment thereof in the compounds of the present invention have binding affinity for PSMA (anti-PSMA antibodies). Prostate-specific membrane antigen (PSMA) is an enzyme that in humans is encoded by the FOLH1 (folate hydrolase 1) gene. Specific binding fragments such as Fab, Fab', F(ab')2 and single-chain specific binding antibodies are typical fragments. In particular, it is preferred that the anti-PSMA antibody is selected from Pelgifatamab, J591 and TLX592 or an antigen-binding fragment thereof. Preferably, the anti-PSMA antibody is selected from Pelgifatamab and J591 or an antigen-binding fragment thereof. More preferably, the anti-PSMA antibody is Pelgifatamab or an antigen-binding fragment thereof.

Pelgifatamab (CAS Registry Number 2414550-93-7, INN published in WHO Recommended List 86, WHO Drug Information, Vol.35, No-3, 2021) is a monoclonal antibody having binding affinity for the antigen PSMA (anti-PSMA antibody). Its preparation, isolation and purification has been described in e.g., US8114965 (Pelgifatamab=clone006).

Pelgifatamab comprises a heavy chain nucleotide sequence according to SEQ ID NO. 1:
SEQ ID NO. 1 - Heavy Chain 
caggtgcagctcgtggaatcgggggggggcgtggtccagccggggcggtcgctccgcctctcgtgcgcagcctcgggcttcgc
cttcagccgctacggcatgcactgggtccgccaggcgcccggcaagggcctggagtgggtcgcggtcatctggtacgacgggt
ccaacaagtattatgcagactccgtcaagggtcggttcaccatcagccgggacaactccaagaacacccagtacctgcagatg
aacagccttcgagcggaggacaccgcggtctactactgcgcccggggcggggatttcctgtactactactactatggcatggacg
tctggggccagggtaccaccgtgaccgtatcctccgcatccactaagggcccgtccgttttcccgctggcaccttcgtcgaagagc
acgtccgggggcacagccgcgctgggctgcctcgtgaaggactactttccggagcccgtcaccgtgtcatggaactcgggcgc
gctcacgtccggcgtccacacgttcccggccgtattgcagagctcgggactctactccctgagctcggtcgtcaccgtgccctcctc
gagcctcggcacccagacctacatctgcaacgtgaaccacaaaccctccaatacgaaggtggacaagcgcgtcgaaccgaa
gtcgtgcgacaagacccacacgtgcccgccgtgcccggcaccggagctgctcggcgggccctcggtcttcctgttcccgccgaa
gcccaaggacaccctgatgatctcgcgcaccccggaggtcacatgcgtggtcgtggacgtgtcccacgaagacccggaggtc
aagttcaactggtacgtcgacggcgtcgaggtccacaacgcgaagaccaagccgcgggaggagcagtacaacagcacgta
ccgcgtcgtttccgtcctcaccgtcttgcaccaggactggctgaacggcaaggagtacaagtgtaaggtgtccaacaaggccttg
cccgcccccattgagaagaccatcagcaaggccaagggccagccccgcgagccgcaggtctacaccctgcccccgtcgcgc
gaagaaatgacgaagaaccaggtctccctgacctgcctcgtcaaggggttctacccgtcggacatcgcagtggagtgggagtc
gaacggccagcccgagaataactacaagacgacccccccagtcctggactccgacggcagcttcttcctttactccaagctgac
cgtcgacaagtcgcggtggcagcagggcaacgtcttctcgtgctcggtgatgcacgaggcgctgcacaaccactacacccaga
agtccctctcgctgtcgccgggcaagtga,
 and a light chain nucleotide sequence according to SEQ ID NO. 2:
SEQ ID NO. 2 - Light Chain

gacatccagatgacccagagccccagctccctgtccgcgtcggtcggagaccgggtcaccatcacctgccgcgcgtcgcagg
gcatctcgaactacctggcgtggtaccagcagaagaccggcaaggtgccgaagttccttatctacgaggcctctacgctccagtc
gggagtcccgtcgcggttcagcgggggtggctccgggaccgacttcacgctgaccatctcgagtctccagccggaggacgtcg
cgacctattactgtcagaactacaactcggccccccttcaccttcggcccgggcaccaaggtcgacatcaagcgcaccgtcgcgg
cgccgtccgtcttcatcttccccccgtcggacgagcagctcaagtccggcaccgcctcggtcgtgtgcctgctcaacaacttctacc
cccgagaggcaaaggtgcagtggaaggtcgacaacgccctgcagtccggcaactcgcaggagagcgtcacggagcagga
cagcaaggacagtacctacagcctctcctccaccctgaccctgagtaaagccgactacgagaagcacaaggtgtacgcgtgc
gaagtcacccaccagggcctctcgtcccccgtgaccaagtccttcaaccggggcgagtgctga.

Further, Pelgifatamab comprises a heavy chain amino acid sequence according to SEQ ID NO. 3:
SEQ ID NO. 3 - Heavy Chain 
QVQLVESGGG VVQPGRSLRL SCAASGFAFS RYGMHWVRQA PGKGLEWVAV
IWYDGSNKYY ADSVKGRFTI SRDNSKNTQY LQMNSLRAED TAVYYCARGG
DFLYYYYYGM DVWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PG(K)
 and a light chain amino acid sequence according to SEQ ID NO. 4:
SEQ ID NO. 4 - Light Chain

DIQMTQSPSS LSASVGDRVT ITCRASQGIS NYLAWYQQKT GKVPKFLIYE
ASTLQSGVPS RFSGGGSGTD FTLTISSLQP EDVATYYCQN YNSAPFTFGP
GTKVDIKRTV AAPSVFIFPP SDEQLKSGTA SVVCLLNNFY PREAKVQWKV
DNALQSGNSQ ESVTEQDSKD STYSLSSTLT LSKADYEKHK VYACEVTHQG
LSSPVTKSFN RGEC

Further, Pelgifatamab comprises a heavy chain nucleotide sequence according to the sequence shown in Figure 1 and a light chain nucleotide sequence according to the sequence shown in Figure 2.

Further, Pelgifatamab comprises a heavy chain amino acid sequence according to the sequence shown in Figure 1 and a light chain amino acid sequence according to the sequence shown in Figure 2.

Further, Pelgifatamab comprises at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10:
Pelgifatamab CDR Heavy Chain Sequences
SEQ ID NO. 5: RYGMH
SEQ ID NO. 6: VIWYDGSNKYYADSVKG
SEQ ID NO. 7: GGDFLYYYYYGMDV
Pelgifatamab CDR Light Chain Sequences
SEQ ID NO. 8: RASQGISNYLA
SEQ ID NO. 9: EASTLQS
SEQ ID NO. 10: QNYNSAPFT

Further, Pelgifatamab comprises at least the three CDR heavy chain sequences and the three CDR light chain sequences according to the sequences shown in Figure 3.

J591 is a monoclonal antibody having binding affinity for the antigen PSMA (anti-PSMA antibody). Its preparation, isolation and purification has been described e.g., in WO2002/098897 and EP2277542.

TLX592 is a monoclonal antibody having binding affinity for the antigen PSMA (anti-PSMA antibody) developed by Telix Pharmaceuticals which has been described in e.g., WO2021/000018.

In a further aspect, the present invention relates to a process for the preparation of a tissue-targeting compound of formula (I) 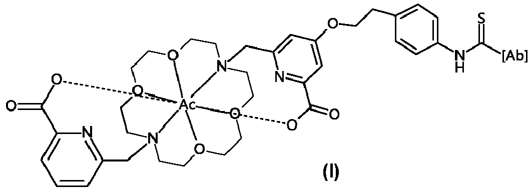 wherein [Ab] is a monoclonal antibody or an antigen-binding fragment thereof having binding affinity for PSMA,
said method comprising
(i) coupling a chelating moiety of formula (II) 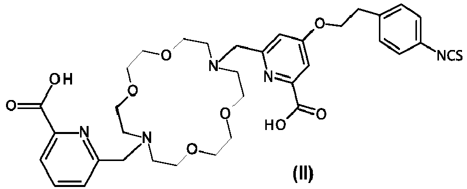 with a monoclonal antibody or an antigen-binding fragment thereof having binding affinity for PSMA, and
(ii) contacting the resulting product with an aqueous solution comprising an ion of at least one alpha-emitting actinium isotope.

In a further embodiment, the present invention relates to a process for the preparation of a tissue-targeting compound of formula (I) 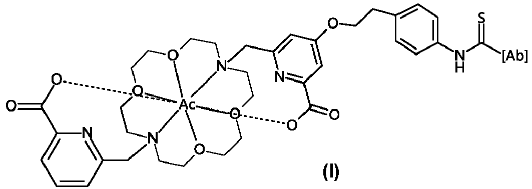 wherein [Ab] is a monoclonal antibody or an antigen-binding fragment thereof having binding affinity for PSMA,
said method comprising
(i) coupling a chelating moiety of formula (II) 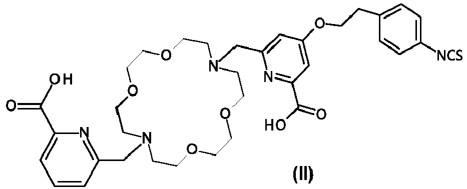 with a monoclonal antibody or an antigen-binding fragment thereof having binding affinity for PSMA via a Lysine residue of the monoclonal antibody or antigen-binding fragment thereof, and
(ii) contacting the resulting product with an aqueous solution comprising an ion of at least one alpha-emitting actinium isotope.

In a further embodiment, the present invention relates to a process for the preparation of a tissue-targeting compound of formula (I) 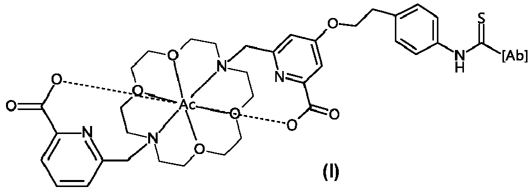 wherein [Ab] is an anti-PSMA antibody selected from Pelgifatamab, J591 and TLX592 or an antigen-binding fragment thereof,
said method comprising
(i) coupling a chelating moiety of formula (II) 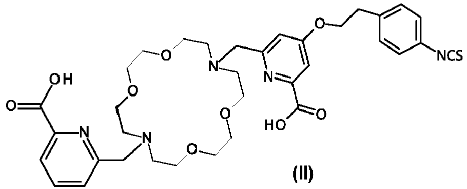 with an anti-PSMA antibody selected from Pelgifatamab, J591 and TLX592 or an antigen-binding fragment thereof via a Lysine residue of the anti-PSMA antibody or antigen-binding fragment thereof, and
(ii) contacting the resulting product with an aqueous solution comprising an ion of at least one alpha-emitting actinium isotope.

In a further embodiment, the present invention relates to a process for the preparation of a tissue-targeting compound of formula (I) 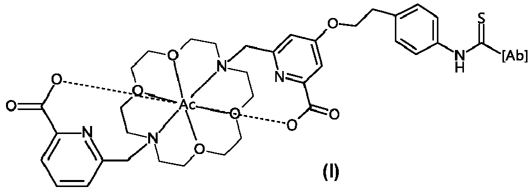 wherein [Ab] is the anti-PSMA antibody Pelgifatamab or an antigen-binding fragment thereof, said method comprising
(i) coupling a chelating moiety of formula (II) 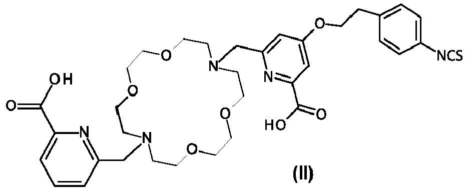 with the anti-PSMA antibody Pelgifatamab or an antigen-binding fragment thereof via a Lysine residue of the monoclonal antibody or antigen-binding fragment thereof, and
(ii) contacting the resulting product with an aqueous solution comprising an ion of at least one alpha-emitting actinium isotope.

In step (i), a chelating moiety of formula (II) 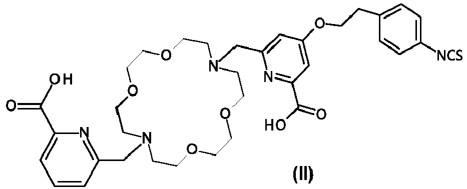 is coupled with a monoclonal antibody or an antigen-binding fragment thereof having binding affinity for PSMA. Preferably, the anti-PSMA antibody is selected from Pelgifatamab, J591 and TLX592 or an antigen-binding fragment thereof. More preferably, the anti-PSMA antibody is Pelgifatamab or an antigen-binding fragment thereof.

Preferably, the chelating moiety of formula (II) is coupled to the anti-PSMA antibody or an antigen-binding fragment thereof via a Lysine residue of the monoclonal antibody, thus forming a thiourea moiety that links the chelating moiety with the anti-PSMA antibody or antigen-binding fragment thereof.

Preferably, the coupling in step (i) is carried out under basic pH, i.e., at a pH higher than 7. More preferably, the coupling in step (i) is carried out at a pH of from 8 to 10, most preferably at a pH of 9.

After step (i), a tissue-targeting chelator of formula (III) is generated. Thus, in further embodiments, the present invention relates to a tissue-targeting chelator of formula (III) 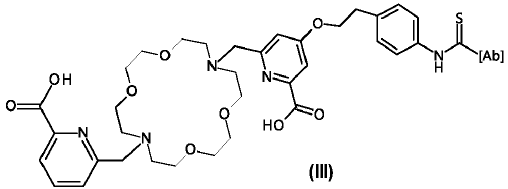 wherein [Ab] is a monoclonal antibody or an antigen-binding fragment thereof having binding affinity for PSMA.

Preferably, the anti-PSMA antibody or antigen-binding fragment thereof is selected from Pelgifatamab, J591 and TLX592 or an antigen-binding fragment thereof. More preferably, the anti-PSMA antibody is Pelgifatamab or an antigen-binding fragment thereof.

In step (ii), the product resulting from the coupling in step (i) is contacted with an aqueous solution comprising an ion of at least one alpha-emitting actinium isotope. Preferably, the alpha-emitting actinium isotope is 225Ac. Preferably, the ion of at least one alpha-emitting actinium isotope is ²²⁵Ac having a triple positive charge, i.e., ²²⁵Ac³⁺. The present invention allows for the execution of step (ii) at room temperature, i.e., at a temperature that is lower than the one required for the preparation of the corresponding DOTA conjugates (i.e. Ac225-DOTA-Pelgifatamab), which require higher temperatures (60°C).

In a further aspect, the tissue-targeting compounds of formula (I) of the present invention may be used for the treatment of disease in a human or in a non-human animal subject. In particular, the tissue-targeting compounds of formula (I) of the present invention may be used for the treatment of hyperplastic and/or neoplastic diseases, such as cancer. There are a number of targets which are known to be associaated with hyperplastic and neoplastic disease. These include certain receptors, cell surface proteins, transmembrane proteins and proteins/peptides found in the extracellular matrix in the vicinity of diseased cells. Examples of cell-surface receptors and antigens which may be associated with neoplastic disease include but are not limited to CD22, CD33, FGFR2 (CD332), PSMA, HER2 and Mesothelin. In one preferred embodiment, the tissue-targeting compounds of formula (I) of the present invention comprise a tissue-targeting moiety comprising a monoclonal antibody having specificity and/or binding affinity for PSMA. This may be reflected, for example by having 50 or more times greater binding affinity for cells expressing PSMA than for non-PSMA expressing cells (preferably at least 100 time greater, more preferably at least 300 times greater). It is believed that PSMA is expressed and/or over-expressed in cells having certain disease states (as indicated herein) and thus the PSMA specific binder may serve to target the complex to such disease-affected cells. Similarly, a tissue targeting moiety may bind to cell-surface markers (e.g. PSMA receptors) present on cells in the vicinity of disease affected cells. PSMA cell-surface markers may be more heavily expressed on diseased cell surfaces than on healthy cell surfaces or more heavily expressed on cell surfaces during periods of growth or replication than during dormant phases. In one embodiment, a PSMA-specific tissue-targeting binder may be used in combination with another binder for a disease-specific cell-surface marker, thus giving a dual-binding complex. Tissue-targeting binders for PSMA will typically be peptides or proteins, as discussed herein.

The various aspects of the invention as described herein relate to treatment of disease, particularly for the selective targeting of diseased tissue, as well as relating to complexes, conjugates, medicaments, formulation, kits etc. useful in such methods. In all aspects, the diseased tissue may reside at a single site in the body (for example in the case of a localized solid tumor) or may reside at a plurality of sites (for example where several joints are affected in arthritis or in the case of a distributed or metastasized cancerous disease).

The diseased tissue to be targeted may be at a soft tissue site, at a calcified tissue site or a plurality of sites which may all be in soft tissue, all in calcified tissue or may include at least one soft tissue site and/or at least one calcified tissue site. In one embodiment, at least one soft tissue site is targeted. The sites of targeting and the sites of origin of the disease may be the same, but alternatively may be different (such as where metastatic sites are specifically targeted). Where more than one site is involved, this may include the site of origin or may be a plurality of secondary sites.

The term "soft tissue" is used herein to indicate tissues which do not have a "hard" mineralized matrix. In particular, soft tissues as used herein may be any tissues that are not skeletal tissues. Correspondingly, "soft tissue disease" as used herein indicates a disease occurring in a "soft tissue" as used herein. The invention is particularly suitable for the treatment of cancers and "soft tissue disease" thus encompasses carcinomas, sarcomas, myelomas, leukemias, lymphomas and mixed type cancers occurring in any "soft" (i.e. non-mineralized) tissue, as well as other non-cancerous diseases of such tissue. Cancerous "soft tissue disease" includes solid tumors occurring in soft tissues as well as metastatic and micro-metastatic tumors. Indeed, the soft tissue disease may comprise a primary solid tumor of soft tissue and at least one metastatic tumor of soft tissue in the same patient. Alternatively, the "soft tissue disease" may consist of only a primary tumor or only metastases with the primary tumor being a skeletal disease. Particularly suitable for treatment and/or targeting in all appropriate aspects of the invention are hematological neoplasms and especially neoplastic diseases of lymphoid cells, such as lymphomas and lymphoid leukemias, including Non-Hodgkin's Lymphoma, B-cell neoplasms of B-cell lymphomas. Similarly, any neoplastic diseases of bone marrow, spine (especially spinal cord) lymph nodes and/or blood cells are suitable for treatment and/or targeting in all appropriate aspects of the invention.

Some examples of B-cell diseases (e.g. neoplasms) that are suitable for treatment and/or targeting in appropriate aspects of the present invention include:
Chronic lymphocytic leukemia/Small lymphocytic lymphoma, B-cell prolymphocytic leukemia, Lymphoplasmacytic lymphoma (such as Waldenstrom macroglobulinemia), Splenic marginal zone lymphoma, Plasma cell neoplasms (e.g. Plasma cell myeloma, Plasmacytoma, Monoclonal immunoglobulin deposition diseases, Heavy chain diseases), Extranodal marginal zone B cell lymphoma (MALT lymphoma), Nodal marginal zone B cell lymphoma (NMZL), Follicular lymphoma, Mantle cell lymphoma, Diffuse large B cell lymphoma, Mediastinal (thymic) large B cell lymphoma, Intravascular large B cell lymphoma, Primary effusion lymphoma and Burkitt lymphoma/leukemia.

The tissue-targeting compounds of formula (I) of the present invention preferably comprise a tissue-targeting moiety comprising a monoclonal antibody having binding affinity for the prostate specific membrane antigen (PSMA). PSMA expression in tumor cells has been observed in different types of diseases, in particular hyperproliferative diseases such as cancer (Uijen et al., Eur. J. Nucl. Med. Mol. Imaging 2021, 48, 4350, doi: 10.1007/s00259-021-05433-w). Examples of diseases and/or neoplasms suitable for treatment using the preferred tissue-targeting compounds of formula (I) of the present invention include prostate cancer, salivary gland cancer, glioblastoma, brain cancer, thyroid cancer, hepatocellular carcinoma and clear cell renal carcinoma.

In some embodiments, the present invention includes a method of using a tissue-targeting compound of formula (I) for the treatment of diseases, more preferably hyperproliferative diseases, more particularly wherein the hyperproliferative disease is cancer, and yet even more particularly wherein the cancer disease is prostate cancer. In some embodiments, the present invention includes a method of using a tissue-targeting compound of formula (I) for the treatment of diseases, preferably hyperproliferative diseases, more preferably cancer, characterized by an overexpression of PSMA in the tumor cells.

In some embodiments, the present invention includes a method of treating a hyperproliferative disease, more particularly cancer, more particularly prostate cancer, said method comprising administering an effective amount of at least one tissue-targeting compound of formula (I) to a subject in need thereof. In some embodiments, the present invention includes a method of treating a hyperproliferative disease characterized by an overexpression of PSMA in the tumor cells, more particularly wherein the hyperproliferative disease is cancer, more particularly prostate cancer, said method comprising administering an effective amount of at least one tissue-targeting compound of formula (I) to a subject in need thereof.

In some embodiments, the present invention provides a tissue-targeting compound of formula (I), as described *supra,* for use in the treatment and/or prophylaxis of diseases, in particular hyperproliferative disorders, more particularly cancer, even more particularly wherein the cancer disease is prostate cancer. In some embodiments, the present invention includes a tissue-targeting compound of formula (I), as described *supra,* for use in the treatment and/or prophylaxis of hyperproliferative diseases characterized by an overexpression of PSMA in the tumor cells.

In some embodiments, the present invention includes a tissue-targeting compound of formula (I) for use in a method of inhibiting proliferation of a cell and/or the induction of apoptosis in a cell, comprising contacting the cell with a tissue-targeting compound of formula (I). In some embodiments, the present invention includes a tissue-targeting compound of formula (I) for use in a method of inhibiting proliferation of tumor cells and/or the induction of apoptosis in tumor cells, comprising contacting the tumor cell with a tissue-targeting compound of formula (I), wherein PSMA is overexpressed in the tumor cells.

In some embodiments, the present invention includes a tissue-targeting compound of formula (I) for use in a method of treating a hyperproliferative disease, more particularly wherein the hyperproliferative disease is cancer, and yet even more particularly wherein the cancer disease is prostate cancer. In some embodiments, the present invention includes a tissue-targeting compound of formula (I) for use in a method of treating a hyperproliferative disease characterized by an overexpression of PSMA in the tumor cells.

In some embodiments, the present invention includes the use of a tissue-targeting compound of formula (I) for the manufacture of a medicament for the treatment and/or prophylaxis of a hyperproliferative disease. In some embodiments, the present invention includes the use of a tissue-targeting compound of formula (I) for the manufacture of a medicament for the treatment and/or prophylaxis of a hyperproliferative disease characterized by an overexpression of PSMA in the tumor cells.

In some embodiments, the present invention includes the use of a tissue-targeting compound of formula (I) for the manufacture of a medicament for the treatment of a hyperproliferative disease, particularly cancer and more particularly prostate cancer.

### DESCRIPTION OF THE FIGURES

Figure 1 Heavy Chain Nucleotide Sequence (SEQ ID NO. 1) and the Heavy Chain Deduced Amino Acid Sequence (SEQ ID NO. 2) for Pelgifatamab (lgG1 Heavy chain cDNA Gene).
Figure 2 Light Chain Nucleotide Sequence (SEQ ID NO. 3) and the Light Chain Deduced Amino Acid Sequence (SEQ ID NO. 4) for Pelgifatamab (kappa Light chain cDNA Gene).
Figure 3. CDR heavy chain sequences (SEQ ID NO. 5, SEQ ID NO. 6 and SEQ ID NO. 7) and CDR light chain sequences (SEQ ID NO. 8, SEQ ID NO. 9 and SEQ ID NO. 10) for Pelgifatamab.

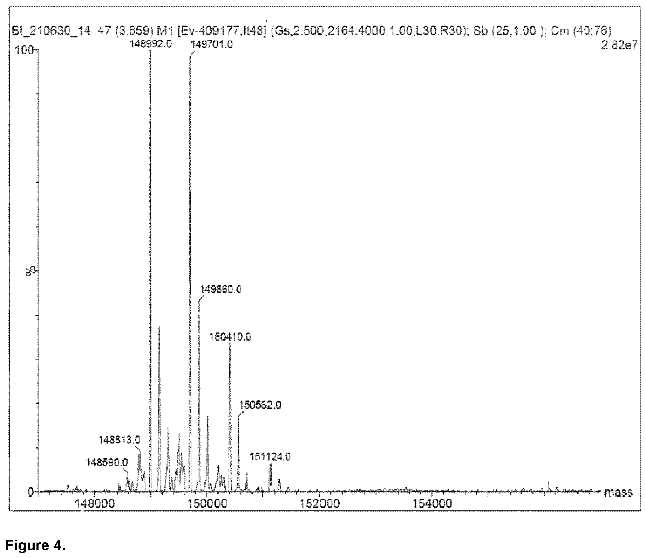

Figure 4. Electrospray mass spectrum of macropa-Pelgifatamab ACC (antibody-chelator conjugate) (CAR 0.8)

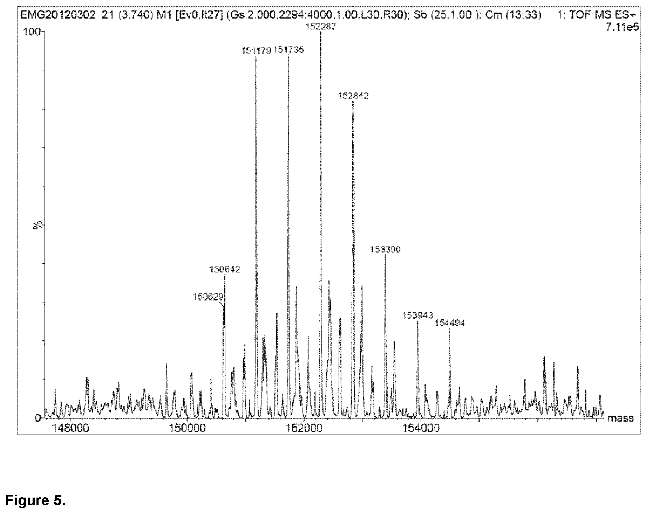

Figure 5. Electrospray mass spectrum of DOTA-Pelgifatamab ACC (antibody-chelator conjugate) (CAR 5.7)

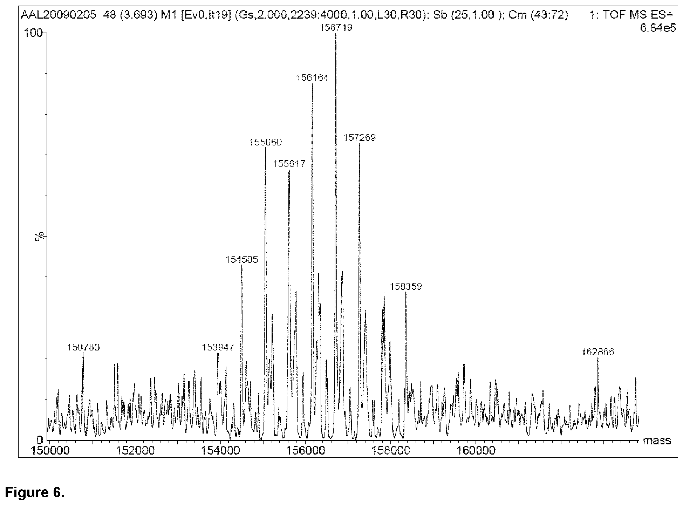

Figure 6. Electrospray mass spectrum of DOTA-Pelgifatamab ACC (antibody-chelator conjugate) (CAR 12.7)
Figure 7. Size-exclusion chromatogram of the targeted actinium conjugates (TACs) Ac225-DOTA-Pelgifatamab (CAR 12.7), Ac225-DOTA-Pelgifatamab (CAR 5.7) and Ac225-Macropa-Pelgifatamab (CAR 0.8).
Figure 8. Immunoreactive Fraction (IRF) of Ac225-Macropa-Pelgifatamab (CAR 0.8), Ac225-DOTA-Pelgifatamab (CAR 5.7) and Ac225-DOTA-Pelgifatamab (CAR 12.7).
Figure 9. Cell cytotoxicity of Ac225-Macropa-Pelgifatamab (CAR 0.8), Ac225-DOTA-Pelgifatamab (CAR 5.7) and Ac225-DOTA-Pelgifatamab (CAR 12.7) against PSMA-expressing prostate cancer cell line C4-2.
Figure 10. Cell cytotoxicity of Ac225-Macropa-Pelgifatamab (CAR 0.8), Ac225-DOTA-Pelgifatamab (CAR 5.7) and Ac225-DOTA-Pelgifatamab (CAR 12.7) against PSMA-expressing prostate cancer cell line MDA-PCa-2b.
Figure 11 depicts the viability of prostate cancer cell lines treated with Ac-225-macropa-Pelgifatamab (CAR 0.8) for 5 days.
Figure 12 depicts the biodistribution of Ac-225-macropa-Pelgifatamab (CAR 0.8) and Ac-225-DOTA-Pelgifatamab (CAR 12.7) in different tissues in the prostate cancer xenograft model MDA-PCA-2b. Animals were dosed with total antibody dose 0.75 mg/kg, 250kBq/kg single dose i.v.
Figure 13 (a) depicts the anti-tumor activity of Ac-225-macropa-Pelgifatamab (CAR 0.8) and Ac-225-DOTA-Pelgifatamab (CAR 12.7) in the prostate cancer xenograft model C4-2 in Balb/c nude male mice, single i.v. injection of 300 kBq/kg, 0.14 mg/kg total antibody (b) depicts body weight changes (in percentage, %) (c) depicts white blood cell counts at the end of the experiment.

### EXPERIMENTAL SECTION

### Conjugation, radiolabeling, purity by SEC, binding (IRF), cell toxicity

Chemical names were generated using the BIOVIA Draw 2019 from Dassault Systèmes. In some cases, generally accepted names of commercially available reagents were used in place of BIOVIA Draw generated names.

The following Table 1 lists the abbreviations used in this paragraph and in the Examples section as far as they are not explained within the text body. Other abbreviations have their meanings customary *per se* to the skilled person.

### Table 1. Abbreviations

The following table lists the abbreviations used herein.
- ²²⁵Ac: actinium-225
- Ac-225: actinium-225
- ACC: antibody-chelator conjugate
- ACN: acetonitrile
- BCA: Bicinchoninic acid
- BRFF-HPC1: Serum free medium
- CAR: chelator-to-antibody ratio
- DCTA: 1,2-diaminocyclohexanetetraacetic acid
- DMA: N,N-dimethylacetamide
- DMSO: dimethyl sulfoxide
- DOTA: 1,4,7,10-tetraazacyclododecane-1,4,7,10-tetraacetic acid
- ESI: electrospray ionization
- EtOH: ethanol
- FBS: fetal bovine serum
- FPLC: fast protein liquid chromatography
- HCI: hydrochloric acid
- HPGe: high purity germanium
- HPLC: high performance liquid chromatography
- iTLC: instant thin layer chromatography
- IRF: immunoreactive fraction
- mAb: monoclonal antibody
- MEM-NEAA: minimum essential medium-non-essential amino acids
- min: minutes
- MS: mass spectrometry
- NaCl: sodium chloride
- nm: nanometer
- nmol: nanomol
- Pelgifatamab: prostate-specific membrane antigen IgG1 antibody
- PBS: phosphate buffered saline
- PSMA: prostate-specific membrane antigen
- RAC: radioactive concentration
- RCP: radiochemical purity
- RPMI: Roswell Park Memorial Institute 1640 medium
- SEC: size exclusion chromatography
- TAC: targeted actinium conjugate (Ac-225 labelled ACC)
- TFA: trifluoroacetic acid
- TOF: time of flight
- UPLC: ultra performance liquid chromatography
- UV: ultraviolet

All reagents, for which the synthesis is not described in the experimental part, are either commercially available, or known compounds or may be formed from known compounds by known methods by a person skilled in the art.

The various aspects of the invention described in this application are illustrated by the following examples which are not meant to limit the invention in any way.

The example testing experiments described herein serve to illustrate the present invention and the invention is not limited to the examples given.

### ACC characterization by size exclusion chromatography and mass spectrometry

CAR of the ACCs was determined by SEC-MS (Water Acquity HPLC connected to Waters XEVO TOF; running buffer: 50/50/0.1 (v/v/v) ACN/Water/TFA; flow rate: 0.06 mL/min, column for on-line desalting: Waters Acquity BEH SEC, 1.7 µm, 2.1 x 150 mm; ESI+ mode) by using MS peak heights in percentage of major peak height for the components mAb, mAb + 1 chelator, mAb + 2 chelators, mAb + 3 chelators etc. and using the formula CAR = Sum(n*An)/Sum An, where n equals the number of chelators and An equals the intensity of the antibody conjugate with n chelators. Purity and concentration of the ACC were determined by SEC-UV (Agilent 1260 Infinity HPLC system, running buffer: 10% DMSO/PBS; flow rate: 0.3 mL/min, column: Waters Acquity BEH SEC, 1.7 µm, 4.6 x 300 mm, detection: UV at 280 nm). The method separates higher aggregate, dimer and fragments from the ACC monomer.

### Example 1: Preparation of macropa-Pelgifatamab ACC

6-[[16-[(6-carboxy-2-pyridyl)methyl]-1,4,10,13-tetraoxa-7,16-diazacyclooctadec-7-yl]methyl]-4-[2-(4-isothiocyanatophenyl)ethoxy]pyridine-2-carboxylic acid (5.8 mg, 8,2 µmol) dissolved in DMA (728 µL) was added to Pelgifatamab (493 mg, 3.3 µmol) in PBS (25 mL). Solution was adjusted to pH 9 by adding 1M carbonate buffer, pH 9.5, (5 mL) and solution shaken overnight. Product was purified by FPLC (column: HiLoad 16/600 Superdex 200 pg column; running buffer: 100 mM acetate/100 mM NaCl 1:1, pH 5; flow: 1 mL/min; detection: UV 214/254 nm) to afford 386 mg (77% yield) of macropa-Pelgifatamab ACC in 100 mM acetate/100 mM NaCl 1:1 (2.3 mg/mL). CAR was determined by MS to be 0.8. Monomeric purity was determined by SEC-UV to be 99%.

### Example 2: Preparation of DOTA-Pelgifatamab ACC CAR 5.7

p-SCN-Bn-DOTA (Macrocyclics, B-205) was dissolved in PBS to 10 mg/mL. 261 µL was added to 990 µL Pelgifatamab (10 mg/mL) dissolved in PBS. The pH was adjusted to 9 with 1M carbonate buffer. The mixture was incubated on a thermomixer shaking at 550 rpm, 37 °C for 2 hours. The conjugate was purified by FPLC (column: HiLoad 16/600 Superdex 200 pg column; running buffer: 100 mM acetate/100 mM NaCl 1:1, pH 5; flow: 1 mL/min; detection: UV 280 nm). The concentration of the combined DOTA-Pelgifatamab fractions was measured by SEC-UV to be 2.6 mg/mL. CAR was determined by MS to be 5.7. Monomeric purity was determined by SEC-UV to be 99%.

### Example 3: Preparation of DOTA-Pelgifatamab ACC CAR 12.7

p-SCN-Bn-DOTA (Macrocyclics, B-205) was dissolved in PBS to 10 mg/mL. 470 µL was added to 990 µL Pelgifatamab (10 mg/mL) dissolved in PBS. The pH was adjusted to 9 with 1M carbonate buffer. The mixture was incubated on a thermomixer shaking at 550 rpm at 22 °C in the dark overnight. The conjugate was purified by FPLC (column: HiLoad 16/600 Superdex 200 pg column; running buffer: 100 mM acetate/100 mM NaCl 1:1, pH 5; flow: 1 mL/min; detection: UV 280 nm). The concentration of the combined DOTA-Pelgifatamab fractions was measured by BCA protein assay to be 2.5 mg/mL. CAR was determined by MS to be 12.7. Monomeric purity was determined by SEC-UV to be 98%.

### Radiolabeling

Macropa-Pelgifatamab, DOTA-Pelgifatamab CAR = 5.7 and DOTA-Pelgifatamab CAR = 12.7 were radiolabeled with Ac-225 at a specific activity of 2 MBq/mg and diluted with 0.1 M acetate buffer pH 5 to a radioactive concentration of 1.5 kBq/µL. The mixture was incubated for one hour at room temperature for macropa-Pelgifatamab while the two DOTA-Pelgifatamab ACCs were incubated for one hour at 60 °C on a heating block. The RCP of the TACs was measured by instant thin layer chromatography using citrate buffer for elution, three replicates for each sample. Unlabeled Ac-225 elutes in the solvent front while the radiolabeled compounds do not move from the application section. The measurement of radioactivity was done with a Digital Gamma-Ray Spectrometer (DSPEC-50, Ortec). The activities were measured at least 6 hours after radiolabeling to allow daughter nuclides to be in equilibrium with Ac-225. The RCP of the samples (defined as [(radioactivity of application section)/(total radioactivity on strip)] x 100) was 99.9% ± 0.013; 99.2% ± 0.049 and 99.5% ± 0.110 for Ac-225-macropa-Pelgifatamab, Ac-225-DOTA-Pelgifatamab CAR 5.7 and Ac-225-DOTA-Pelgifatamab CAR 12.7, respectively.

### Size Exclusion Chromatography of TACs

Purity of the TACs were determined by SEC-UV. The method separates higher aggregate, dimer and fragments from the ACC monomer. The separation was done using a Acquity UPLC Protein BEH SEC, 1.7 µm, 200Å, 4.6 x 300 mm column operated at 30 °C. The mobile phase was 170 mM Ammonium acetate and 300 mM NaCl containing 10% 2-propanol and 0.1 mM DCTA. The flowrate was 0.3 mL/min, and the ACCs were detected at UV 280 nm. The analysis was performed on an Agilent 1200 series HPLC with 35 µL injections.

**Table 2. Purity of TACs by SEC-UV**

| TAC | Area % (UV) | | |
|---|---|---|---|
| | Aggregate | Dimer | Monomer |
| Ac-225-macropa-Pelgifatamab | ND | 0.5 | 99.5 |
| Pelgifatamab-DOTA-Ac-225 CAR 5.7 | 18.4 | 3.0 | 78.6 |
| Pelgifatamab-DOTA-Ac-225 CAR 12.7 | 30.5 | 4.8 | 64.7 |

### Immunoreactive Fraction (IRF)

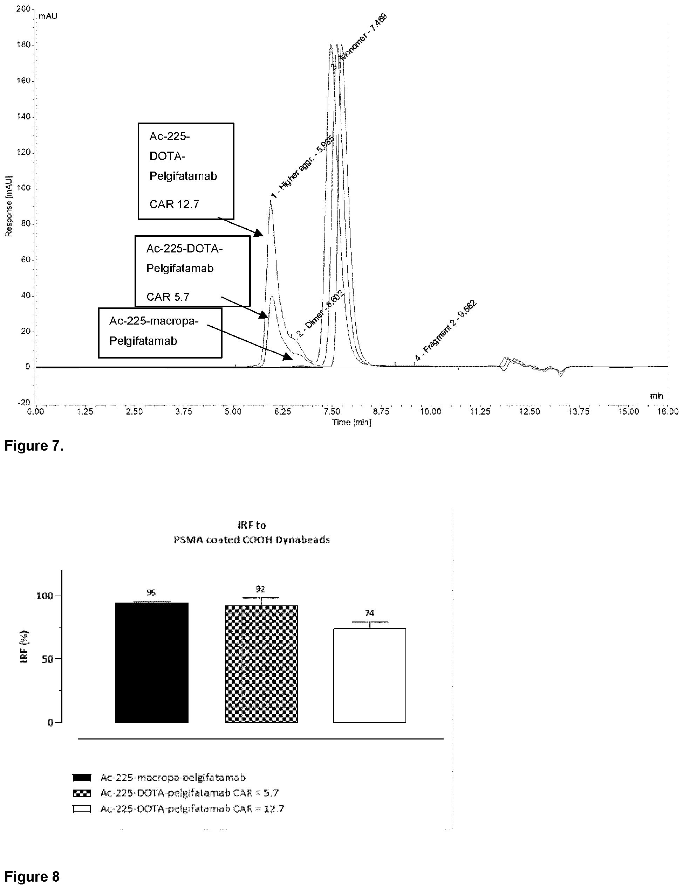

Shortly after completion of the radiolabeling, the binding of each sample to PSMA-coated M-270-carboxy Dynabeads was determined. In short, 250 µg beads were placed in 2 mL Eppendorf tubes in 50 µL buffer (PBS/3% BSA). The samples were run in triplicate and samples blocked with naked PSMA mAb in excess were included. The blocking step lasted for 40 min, while shaking the tubes on a Thermomixer (Eppendorf) at 750 rpm at room temperature. The radiolabeled compounds were diluted to 5 Bq/µL in buffer and added to each bead-containing tube. The binding lasted for 80 min while shaking at 750 rpm at room temperature. After completion of the binding step, 40 µL of buffer was added to each tube and a magnet (DynaMag-2) was used to separate the beads from half of the supernatant. The radio activities in the supernatant and in the beads of each sample were measured using a Digital Gamma-Ray Spectrometer. The activities were measured after at least 6 hours, to allow daughter nuclides to be in equilibrium with Ac-225. Total binding was defined as [(activity in beads - activity in supernatant)/(total activity)] x 100. The unspecific binding was obtained from the same calculation from blocked samples, and IRF was defined as [specific binding - unspecific binding]. The IRF was 95%, 92% and 74% for Ac-225-macropa-Pelgifatamab, Ac-225-DOTA-Pelgifatamab CAR 5.7 and Ac-225-DOTA-Pelgifatamab CAR 12.7, respectively (Figure 8).

### Cell cytotoxicity

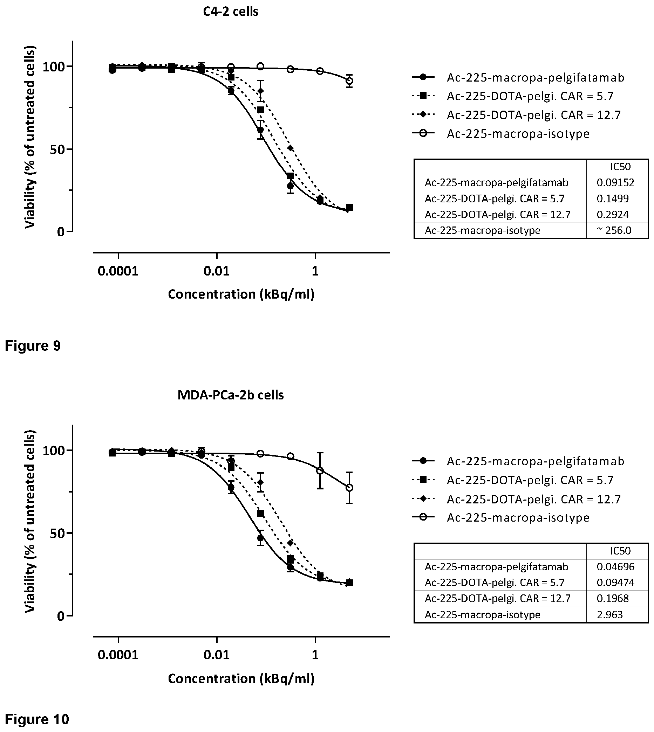

In-vitro cytotoxicity of Ac-225-macropa-Pelgifatamab, Ac-225-DOTA-Pelgifatamab CAR=5.7 and Ac-225-DOTA-Pelgifatamab CAR=12.7 was measured in the PSMA expressing prostate cancer cell lines C4-2 and MDA-PCa-2b. Cells were seeded at an appropriate cell density in cell medium (RPMI/10% FBS/1% HEPES/1% MEM NEAA/1% NaPyruvate/1% L-glutamine and BRFF-HPC1/20% FBS, respectively). One day after seeding, the Ac-225 labeled compounds at a specific activity of 2 MBq/mg were titrated in parallel with a relevant negative isotype control which was radiolabeled in the same conditions. Cells were exposed for two hours to the radioligands. Cell viability was determined six days later using CellTiterGlo (Promega) and luminescence was measured on EnVision plate reader. The IC50 values were 0.09, 0.15 and 0.29 kBq/ml for Ac-225-macropa-Pelgifatamab, Ac-225-DOTA-Pelgifatamab CAR 5.7 and Ac-225-DOTA-Pelgifatamab CAR 12.7, respectively in C4-2 cells (Figure 9) and 0.05, 0.09 and 0.2 kBq/ml for Ac-225-macropa-Pelgifatamab, Ac-225-DOTA-Pelgifatamab CAR 5.7 and Ac-225-DOTA-Pelgifatamab CAR 12.7, respectively in MDA-PCa-2b cells (Figure 10). IC50 values of Ac-225-macropa-isotype were -256 kBq/ml in C4-2 cells and 3 kBq/ml in MDA-PCa-2b cells (Figure 9+10).

### Cell cytotoxicity

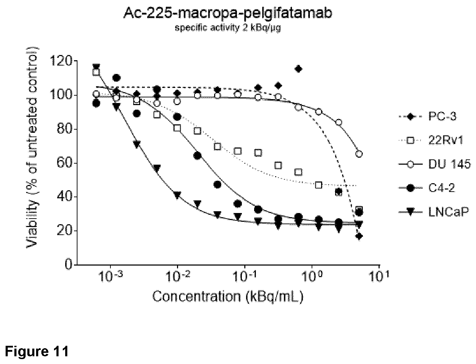

*In-vitro* cytotoxicity of Ac-225-macropa-Pelgifatamab was measured in prostate cancer cell lines expressing different levels of PSMA. PC-3, DU-145, 22Rv1, C4-2 and LNCaP cells were seeded at an appropriate cell density in the following cell media respectively: DMEM/ Ham's F12 + 10% FCS; DMEM + 10% FCS; RPMI + 10% FCS; DMEM/ Ham's F12 + 10% FCS + Insulin (human) (5µg/ml, Sigma #I9278), Transferrin (human); (final: 5µg/ml), D-Biotin (0,25µg/ml final, Sigma:B4639), Adenine (final: 25µg/ml, Sigma: A9795), L-3,3-,5-Triiodothyronine, Sodium Salt, Sigma: T6397 (13.6pg/ml final); RPMI + 20% FCS suggested by the respective suppliers. One day after seeding, Ac-225-macropa-Pelgifatamab at a specific activity of 2 MBq/mg were titrated in parallel with a relevant negative isotype control which was radiolabeled in the same conditions. Cells were treated for 5 days and subsequentially cell viability was determined using CellTiterGlo (Promega). Strong cytotoxicity was observed in LNCaP, C4-2 and 22R1 cells with little cytotoxicity in PMSA negative PC-3 and DU-145 cells (Figure 11)

### Biodistribution study of Ac-225-macropa-Pelgifatamab

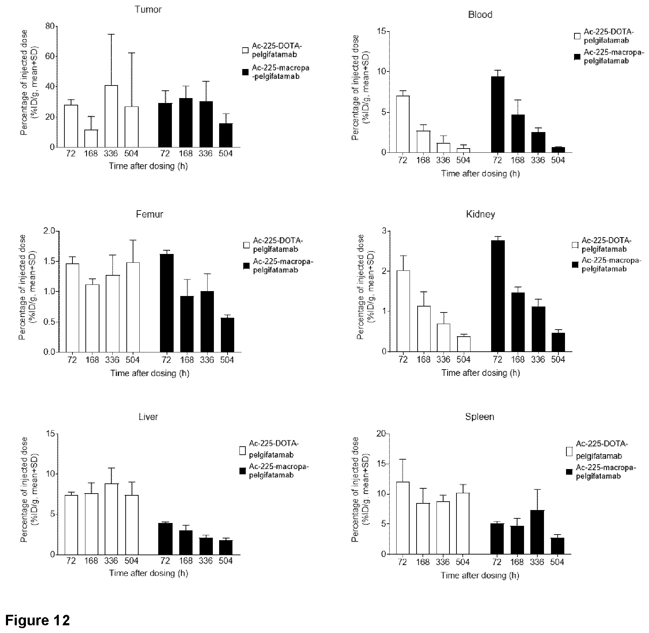

Biodistribution of Ac-225-macropa-Pelgifatamab was investigated in the PSMA-expressing prostate cancer xenograft model MDA PCa 2b. Female RJ:NMRI-Foxn1nu/nu mice were implanted with testosterone pellets subcutaneously and were inoculated with 1 x 10⁶ MDA PCa 2b cells on the right flank. Animals were pre-treated with 200 µg/mouse unspecific IgG2a antibody 24 hours prior to hot dosing. Animals were treated with 300 kBq/kg Ac-225-macropa-Pelgifatamab IV at a total protein dose of 0.75 mg/kg. In a separate *in vivo* study the xenograft model MDA PCa 2b was treated with 250 kBq/kg Ac-225-DOTA-Pelgifatamab at a protein dose of 0.14 mg/kg. In both studies, animals were sacrificed 72, 168, 336, and 504 hours post treatment and 225Ac in blood, tumor, liver, kidney, spleen and femur was determined. For Ac-225-macropa-Pelgifatamab-treated animals, the data indicate strong and persistent tumor accumulation whereas accumulation in normal organs was generally low and transient with clearance over time. Compared with 225Ac-DOTA-Pelgifatamab-treated animals, improved clearance from the bone, lower and over time decreasing radioactivity in the liver and less uptake in the spleen was seen (Figure 12). These data might be explained with an improved *in vivo* complex stability with 225Ac for macropa compared with DOTA.

### In vivo efficacy study comparing Ac-225-macropa-Pelgifatamab and Ac-225-DOTA-Pelgifatamab

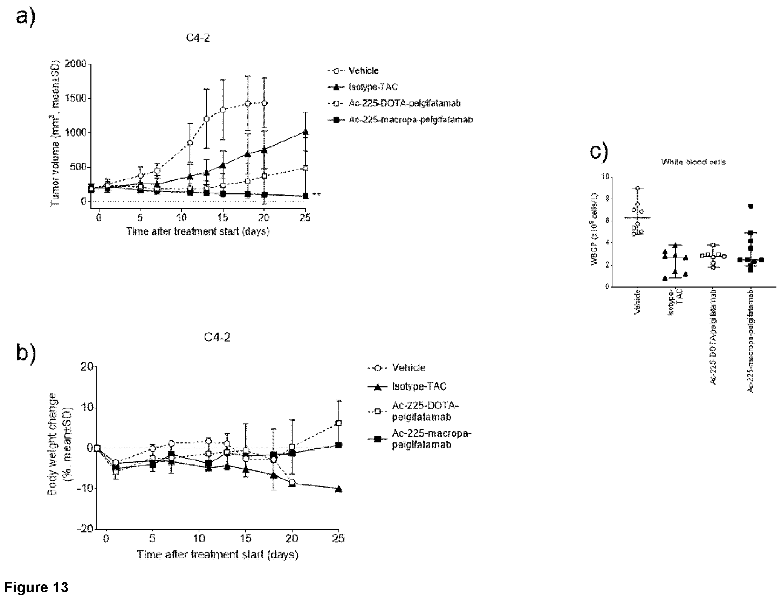

The compounds Ac-225-macropa-Pelgifatamab, Ac-225-DOTA-Pelgifatamab and isotype-macropa were labeled with 225Ac. Mice xenografted with C4-2 prostate cancer cells were treated with a single dose IV of 300 kBq/kg with a total antibody dose of 0.14 mg/kg. As presented in Figure 13 (A) and (B), higher antitumor activity of Ac-225-macropa-Pelgifatamab was seen compared with Ac-225-DOTA-Pelgifatamab. Body weight loss was less than 10% for all targeted treatment groups. Effects on the hematopoietic system were determined by WBC counts at the end of the experiment (Figure 13 (C)).

## Claims

1. A tissue-targeting compound of formula (I) 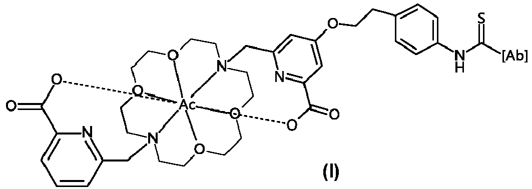 wherein [Ab] is a monoclonal antibody or an antigen-binding fragment thereof having binding affinity for the prostate-specific membrane antigen (PSMA).

2. The tissue-targeting compound of formula (I) according to claim 1, wherein [Ab] is the anti-PSMA-monoclonal antibody Pelgifatamab or an antigen-binding fragment thereof.

3. The tissue-targeting compound of formula (I) according to any of claims 1 or 2, wherein [Ab] is the anti-PSMA antibody Pelgifatamab or an antigen-binding fragment thereof comprising a heavy chain sequence according to SEQ ID NO. 1, and a light chain sequence according to SEQ ID NO. 2.

4. The tissue-targeting compound of formula (I) according to any of claims 1 or 2, wherein [Ab] is the anti-PSMA-antibody Pelgifatamab or an antigen-binding fragment thereof comprising at least the three CDR heavy chain sequences according to SEQ ID NO. 5, SEQ ID NO. 6 and SEQ ID NO. 7 and the three CDR light chain sequences according to SEQ ID NO. 8, SEQ ID NO. 9 and SEQ ID NO. 10.

5. The tissue-targeting compound of formula (I) according to any of claims 1 or 2, wherein [Ab] is the anti-PSMA antibody Pelgifatamab or an antigen-binding fragment thereof comprising a heavy chain sequence according to SEQ ID NO. 1:
SEQ ID NO. 1 - Heavy Chain 
caggtgcagctcgtggaatcgggggggggcgtggtccagccggggcggtcgctccgcctctcgtgcgcagcctcgg
gcttcgccttcagccgctacggcatgcactgggtccgccaggcgcccggcaagggcctggagtgggtcgcggtcatct
 
ggtacgacgggtccaacaagtattatgcagactccgtcaagggtcggttcaccatcagccgggacaactccaagaac
acccagtacctgcagatgaacagccttcgagcggaggacaccgcggtctactactgcgcccggggcggggatttcct
gtactactactactatggcatggacgtctggggccagggtaccaccgtgaccgtatcctccgcatccactaagggccc
gtccgttttcccgctggcaccttcgtcgaagagcacgtccgggggcacagccgcgctgggctgcctcgtgaaggacta
ctttccggagcccgtcaccgtgtcatggaactcgggcgcgctcacgtccggcgtccacacgttcccggccgtattgcag
agctcgggactctactccctgagctcggtcgtcaccgtgccctcctcgagcctcggcacccagacctacatctgcaacg
tgaaccacaaaccctccaatacgaaggtggacaagcgcgtcgaaccgaagtcgtgcgacaagacccacacgtgc
ccgccgtgcccggcaccggagctgctcggcgggccctcggtcttcctgttcccgccgaagcccaaggacaccctgat
gatctcgcgcaccccggaggtcacatgcgtggtcgtggacgtgtcccacgaagacccggaggtcaagttcaactggt
acgtcgacggcgtcgaggtccacaacgcgaagaccaagccgcgggaggagcagtacaacagcacgtaccgcgt
cgtttccgtcctcaccgtcttgcaccaggactggctgaacggcaaggagtacaagtgtaaggtgtccaacaaggccttg
cccgcccccattgagaagaccatcagcaaggccaagggccagccccgcgagccgcaggtctacaccctgccccc
gtcgcgcgaagaaatgacgaagaaccaggtctccctgacctgcctcgtcaaggggttctacccgtcggacatcgcag
tggagtgggagtcgaacggccagcccgagaataactacaagacgacccccccagtcctggactccgacggcagct
tcttcctttactccaagctgaccgtcgacaagtcgcggtggcagcagggcaacgtcttctcgtgctcggtgatgcacgag
gcgctgcacaaccactacacccagaagtccctctcgctgtcgccgggcaagtga,
 and a light chain sequence according to SEQ ID NO. 2:
SEQ ID NO. 2 - Light Chain

gacatccagatgacccagagccccagctccctgtccgcgtcggtcggagaccgggtcaccatcacctgccgcgcgtc
gcagggcatctcgaactacctggcgtggtaccagcagaagaccggcaaggtgccgaagttccttatctacgaggcct
ctacgctccagtcgggagtcccgtcgcggttcagcgggggtggctccgggaccgacttcacgctgaccatctcgagtct
ccagccggaggacgtcgcgacctattactgtcagaactacaactcggccccttcaccttcggcccgggcaccaagg
tcgacatcaagcgcaccgtcgcggcgccgtccgtcttcatcttccccccgtcggacgagcagctcaagtccggcaccg
cctcggtcgtgtgcctgctcaacaacttctacccccgagaggcaaaggtgcagtggaaggtcgacaacgccctgcag
tccggcaactcgcaggagagcgtcacggagcaggacagcaaggacagtacctacagcctctcctccaccctgacc
ctgagtaaagccgactacgagaagcacaaggtgtacgcgtgcgaagtcacccaccagggcctctcgtcccccgtga
ccaagtccttcaaccggggcgagtgctga.

6. A process for the preparation of a tissue-targeting compound of formula (I) 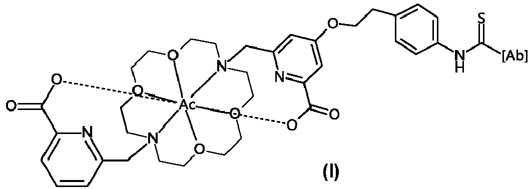 wherein [Ab] is a monoclonal antibody or an antigen-binding fragment thereof having binding affinity for PSMA,
said method comprising
(i) coupling a chelating moiety of formula (II) 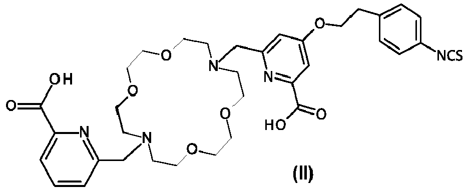 with a monoclonal antibody or an antigen-binding fragment thereof having binding affinity for PSMA, and
(ii) contacting the resulting product with an aqueous solution comprising an ion of at least one alpha-emitting actinium isotope.

7. The process according to claim 6, wherein [Ab] is the anti-PSMA antibody Pelgifatamab or an antigen-binding fragment thereof,
said method comprising
(i) coupling a chelating moiety of formula (II) 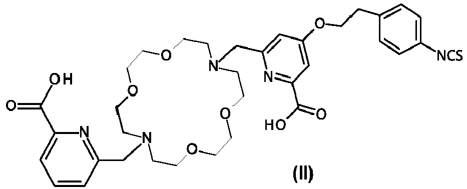 with the anti-PSMA antibody Pelgifatamab or an antigen-binding fragment thereof via a Lysine residue of the monoclonal antibody or antigen-binding fragment thereof, and
(ii) contacting the resulting product with an aqueous solution comprising an ion of at least one alpha-emitting actinium isotope.

8. A tissue-targeting chelator of formula (III) 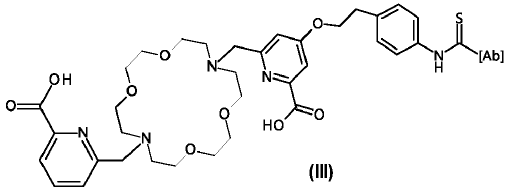 wherein [Ab] is a monoclonal antibody or an antigen-binding fragment thereof having binding affinity for PSMA.

9. Use of a tissue-targeting chelator of formula (III) according to claim 8 for the preparation of a tissue-targeting compound of formula (I) according to any of claims 1 to 5.

10. A tissue-targeting compound of formula (I) according to any of claims 1 to 5 for use in the treatment of disease.

11. The tissue-targeting compound of formula (I) according to any of claims 1 to 5 for use according to claim 10, wherein the disease is **characterized by** an overexpression of the prostate-specific membrane antigen (PSMA).

12. The tissue-targeting compound of formula (I) according to any of claims 1 to 5 for use according to any of claims 10 or 11, wherein the disease is cancer, preferably prostate cancer.
